(19) Europäisches Patentamt
European Patent Office
Office européen des brevets

(11) EP 3 960 879 A1

(12) EUROPEAN PATENT APPLICATION

(43) Date of publication:
**02.03.2022 Bulletin 2022/09**

(21) Application number: **20305967.0**

(22) Date of filing: **01.09.2020**

(51) International Patent Classification (IPC):
***C12R 1/145*** *(2006.01)* ***C12N 9/06*** *(2006.01)*
***C12P 13/06*** *(2006.01)* ***C12R 1/15*** *(2006.01)*
***C12R 1/19*** *(2006.01)*

(52) Cooperative Patent Classification (CPC):
**C12N 9/0016; C07K 14/195; C07K 14/245; C07K 14/39; C07K 14/395; C12N 1/205; C12P 13/06; C12Y 104/01001;** C12R 2001/145; C12R 2001/15; C12R 2001/19

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Metabolic Explorer**
**63360 Saint Beauzire (FR)**

(72) Inventors:
- **DUMON-SEIGNOVERT, Laurence**
  **63430 Pont du Château (FR)**
- **RAYNAUD, Céline**
  **63360 Saint Beauzire (FR)**

(74) Representative: **Regimbeau**
**20, rue de Chazelles**
**75847 Paris Cedex 17 (FR)**

# (54) MICROORGANISM AND METHOD FOR THE IMPROVED PRODUCTION OF ALANINE

(57) The present invention relates to a microorganism genetically modified for improved production of alanine, wherein the microorganism expresses a heterologous *alaD* gene coding an alanine dehydrogenase and has reduced Lrp transcription factor activity and/or expression. The present invention also relates to a method for the production of alanine using said microorganism.

EP 3 960 879 A1

SEQUENCE LISTING

<110> METABOLIC EXPLORER

<120> MICROORGANISM AND METHOD FOR THE IMPROVED PRODUCTION OF ALANINE

<130> B76251EPD40811

<160> 47

<170> PatentIn version 3.5

<210> 1
<211> 164
<212> PRT
<213> Escherichia coli

<400> 1

```
Met Val Asp Ser Lys Lys Arg Pro Gly Lys Asp Leu Asp Arg Ile Asp
1               5                   10                  15

Arg Asn Ile Leu Asn Glu Leu Gln Lys Asp Gly Arg Ile Ser Asn Val
            20                  25                  30

Glu Leu Ser Lys Arg Val Gly Leu Ser Pro Thr Pro Cys Leu Glu Arg
        35                  40                  45

Val Arg Arg Leu Glu Arg Gln Gly Phe Ile Gln Gly Tyr Thr Ala Leu
    50                  55                  60

Leu Asn Pro His Tyr Leu Asp Ala Ser Leu Leu Val Phe Val Glu Ile
65                  70                  75                  80

Thr Leu Asn Arg Gly Ala Pro Asp Val Phe Glu Gln Phe Asn Thr Ala
                85                  90                  95

Val Gln Lys Leu Glu Glu Ile Gln Glu Cys His Leu Val Ser Gly Asp
            100                 105                 110

Phe Asp Tyr Leu Leu Lys Thr Arg Val Pro Asp Met Ser Ala Tyr Arg
        115                 120                 125

Lys Leu Leu Gly Glu Thr Leu Leu Arg Leu Pro Gly Val Asn Asp Thr
    130                 135                 140

Arg Thr Tyr Val Val Met Glu Glu Val Lys Gln Ser Asn Arg Leu Val
145                 150                 155                 160

Ile Lys Thr Arg
```

<210> 2
<211> 495
<212> DNA
<213> Escherichia coli

<400> 2
atggtagata gcaagaagcg ccctggcaaa gatctcgacc gtatcgatcg taacattctt 60

aatgagttgc aaaaggatgg gcgtatttct aacgtcgagc tttctaaacg tgtgggactt 120

tccccaacgc cgtgccttga gcgtgtgcgt cggctggaaa gacaagggtt tattcagggc 180

tatacggcgc tgcttaaccc ccattatctg gatgcatcac ttctggtatt cgttgagatt 240

actctgaatc gtggcgcacc ggatgtgttt gaacaattca ataccgctgt acaaaaactt 300

gaagaaattc aggagtgtca tttagtatcc ggtgatttcg actacctgtt gaaaacacgc 360

gtgccggata tgtcagccta ccgtaagttg ctgggggaaa ccctgctgcg tctgcctggc 420

gtcaatgaca cacggacata cgtcgttatg gaagaagtca agcagagtaa tcgtctggtt 480

attaagacgc gctaa 495

<210> 3
<211> 164
<212> PRT
<213> Artificial Sequence

<220>
<223> Lrp* (L108F)

<400> 3

Met Val Asp Ser Lys Lys Arg Pro Gly Lys Asp Leu Asp Arg Ile Asp
1               5                   10                  15

Arg Asn Ile Leu Asn Glu Leu Gln Lys Asp Gly Arg Ile Ser Asn Val
            20                  25                  30

Glu Leu Ser Lys Arg Val Gly Leu Ser Pro Thr Pro Cys Leu Glu Arg
        35                  40                  45

Val Arg Arg Leu Glu Arg Gln Gly Phe Ile Gln Gly Tyr Thr Ala Leu
    50                  55                  60

Leu Asn Pro His Tyr Leu Asp Ala Ser Leu Leu Val Phe Val Glu Ile
65                  70                  75                  80

Thr Leu Asn Arg Gly Ala Pro Asp Val Phe Glu Gln Phe Asn Thr Ala
                85                  90                  95

Val Gln Lys Leu Glu Glu Ile Gln Glu Cys His Phe Val Ser Gly Asp
            100                 105                 110

```
Phe Asp Tyr Leu Leu Lys Thr Arg Val Pro Asp Met Ser Ala Tyr Arg
        115                 120                 125

Lys Leu Leu Gly Glu Thr Leu Leu Arg Leu Pro Gly Val Asn Asp Thr
    130                 135                 140

Arg Thr Tyr Val Val Met Glu Glu Val Lys Gln Ser Asn Arg Leu Val
145                 150                 155                 160

Ile Lys Thr Arg


<210>  4
<211>  495
<212>  DNA
<213>  Artificial Sequence

<220>
<223>  Lrp* (L108F)

<400>  4
atggtagata gcaagaagcg ccctggcaaa gatctcgacc gtatcgatcg taacattctt      60

aatgagttgc aaaaggatgg gcgtatttct aacgtcgagc tttctaaacg tgtgggactt     120

tccccaacgc cgtgccttga gcgtgtgcgt cggctggaaa gacaagggtt tattcagggc     180

tatacggcgc tgcttaaccc ccattatctg gatgcatcac ttctggtatt cgttgagatt     240

actctgaatc gtggcgcacc ggatgtgttt gaacaattca ataccgctgt acaaaaactt     300

gaagaaattc aggagtgtca ttttgtatcc ggtgatttcg actacctgtt gaaaacacgc     360

gtgccggata tgtcagccta ccgtaagttg ctgggggaaa ccctgctgcg tctgcctggc     420

gtcaatgaca cacggacata cgttgttatg gaagaagtca agcagagtaa tcgtctggtt     480

attaagacgc gctaa                                                      495


<210>  5
<211>  164
<212>  PRT
<213>  Artificial Sequence

<220>
<223>  Lrp* (L74F)

<400>  5

Met Val Asp Ser Lys Lys Arg Pro Gly Lys Asp Leu Asp Arg Ile Asp
1               5                   10                  15

Arg Asn Ile Leu Asn Glu Leu Gln Lys Asp Gly Arg Ile Ser Asn Val
            20                  25                  30

Glu Leu Ser Lys Arg Val Gly Leu Ser Pro Thr Pro Cys Leu Glu Arg
```

35 40 45

Val Arg Arg Leu Glu Arg Gln Gly Phe Ile Gln Gly Tyr Thr Ala Leu
50 55 60

Leu Asn Pro His Tyr Leu Asp Ala Ser Phe Leu Val Phe Val Glu Ile
65 70 75 80

Thr Leu Asn Arg Gly Ala Pro Asp Val Phe Glu Gln Phe Asn Thr Ala
85 90 95

Val Gln Lys Leu Glu Glu Ile Gln Glu Cys His Leu Val Ser Gly Asp
100 105 110

Phe Asp Tyr Leu Leu Lys Thr Arg Val Pro Asp Met Ser Ala Tyr Arg
115 120 125

Lys Leu Leu Gly Glu Thr Leu Leu Arg Leu Pro Gly Val Asn Asp Thr
130 135 140

Arg Thr Tyr Val Val Met Glu Glu Val Lys Gln Ser Asn Arg Leu Val
145 150 155 160

Ile Lys Thr Arg

<210> 6
<211> 495
<212> DNA
<213> Artificial Sequence

<220>
<223> Lrp* (L74F)

<400> 6
atggtagata gcaagaagcg ccctggcaaa gatctcgacc gtatcgatcg taacattctt 60

aatgagttgc aaaaggatgg gcgtatttct aacgtcgagc tttctaaacg tgtgggactt 120

tccccaacgc cgtgccttga gcgtgtgcgt cggctggaaa gacaagggtt tattcagggc 180

tatacggcgc tgcttaaccc ccattatctg gatgcatcat ttctggtatt cgttgagatt 240

actctgaatc gtggcgcacc ggatgtgttt gaacaattca ataccgctgt acaaaaactt 300

gaagaaattc aggagtgtca tttagtatcc ggtgatttcg actacctgtt gaaaacacgc 360

gtgccggata tgtcagccta ccgtaagttg ctgggggaaa ccctgctgcg tctgcctggc 420

gtcaatgaca cacggacata cgttgttatg gaagaagtca agcagagtaa tcgtctggtt 480

attaagacgc gctaa 495

<210> 7
<211> 164
<212> PRT
<213> Artificial Sequence

<220>
<223> Lrp* (F113C)

<400> 7

```
Met Val Asp Ser Lys Lys Arg Pro Gly Lys Asp Leu Asp Arg Ile Asp
1               5                   10                  15

Arg Asn Ile Leu Asn Glu Leu Gln Lys Asp Gly Arg Ile Ser Asn Val
            20                  25                  30

Glu Leu Ser Lys Arg Val Gly Leu Ser Pro Thr Pro Cys Leu Glu Arg
        35                  40                  45

Val Arg Arg Leu Glu Arg Gln Gly Phe Ile Gln Gly Tyr Thr Ala Leu
    50                  55                  60

Leu Asn Pro His Tyr Leu Asp Ala Ser Leu Leu Val Phe Val Glu Ile
65                  70                  75                  80

Thr Leu Asn Arg Gly Ala Pro Asp Val Phe Glu Gln Phe Asn Thr Ala
                85                  90                  95

Val Gln Lys Leu Glu Glu Ile Gln Glu Cys His Leu Val Ser Gly Asp
            100                 105                 110

Cys Asp Tyr Leu Leu Lys Thr Arg Val Pro Asp Met Ser Ala Tyr Arg
        115                 120                 125

Lys Leu Leu Gly Glu Thr Leu Leu Arg Leu Pro Gly Val Asn Asp Thr
    130                 135                 140

Arg Thr Tyr Val Val Met Glu Glu Val Lys Gln Ser Asn Arg Leu Val
145                 150                 155                 160

Ile Lys Thr Arg
```

<210> 8
<211> 495
<212> DNA
<213> Artificial Sequence

<220>
<223> Lrp* (F113C)

<400> 8

atggtagata gcaagaagcg ccctggcaaa gatctcgacc gtatcgatcg taacattctt 60

aatgagttgc aaaaggatgg gcgtatttct aacgtcgagc tttctaaacg tgtgggactt 120

tccccaacgc cgtgccttga gcgtgtgcgt cggctggaaa gacaagggtt tattcagggc 180

tatacggcgc tgcttaaccc ccattatctg gatgcatcac ttctggtatt cgttgagatt 240

actctgaatc gtggcgcacc ggatgtgttt gaacaattca ataccgctgt acaaaaactt 300

gaagaaattc aggagtgtca tttagtatcc ggtgattgcg actacctgtt gaaaacacgc 360

gtgccggata tgtcagccta ccgtaagttg ctggggggaaa ccctgctgcg tctgcctggc 420

gtcaatgaca cacggacata cgttgttatg gaagaagtca agcagagtaa tcgtctggtt 480

attaagacgc gctaa 495

<210> 9
<211> 166
<212> PRT
<213> Artificial Sequence

<220>
<223> Lrp* (123PD)

<400> 9

```
Met Val Asp Ser Lys Lys Arg Pro Gly Lys Asp Leu Asp Arg Ile Asp
1               5                   10                  15

Arg Asn Ile Leu Asn Glu Leu Gln Lys Asp Gly Arg Ile Ser Asn Val
            20                  25                  30

Glu Leu Ser Lys Arg Val Gly Leu Ser Pro Thr Pro Cys Leu Glu Arg
        35                  40                  45

Val Arg Arg Leu Glu Arg Gln Gly Phe Ile Gln Gly Tyr Thr Ala Leu
    50                  55                  60

Leu Asn Pro His Tyr Leu Asp Ala Ser Leu Leu Val Phe Val Glu Ile
65                  70                  75                  80

Thr Leu Asn Arg Gly Ala Pro Asp Val Phe Glu Gln Phe Asn Thr Ala
                85                  90                  95

Val Gln Lys Leu Glu Glu Ile Gln Glu Cys His Leu Val Ser Gly Asp
            100                 105                 110

Phe Asp Tyr Leu Leu Lys Thr Arg Val Pro Asp Pro Asp Met Ser Ala
        115                 120                 125

Tyr Arg Lys Leu Leu Gly Glu Thr Leu Leu Arg Leu Pro Gly Val Asn
    130                 135                 140
```

Asp Thr Arg Thr Tyr Val Val Met Glu Glu Val Lys Gln Ser Asn Arg
145 150 155 160

Leu Val Ile Lys Thr Arg
165

<210> 10
<211> 501
<212> DNA
<213> Artificial Sequence

<220>
<223> Lrp* (123PD)

<400> 10
atggtagata gcaagaagcg ccctggcaaa gatctcgacc gtatcgatcg taacattctt 60

aatgagttgc aaaaggatgg gcgtatttct aacgtcgagc tttctaaacg tgtgggactt 120

tccccaacgc cgtgccttga gcgtgtgcgt cggctggaaa gacaagggtt tattcagggc 180

tatacggcgc tgcttaaccc ccattatctg gatgcatcac ttctggtatt cgttgagatt 240

actctgaatc gtggcgcacc ggatgtgttt gaacaattca ataccgctgt acaaaaactt 300

gaagaaattc aggagtgtca tttagtatcc ggtgatttcg actacctgtt gaaaacacgc 360

gtgccggatc cggatatgtc agcctaccgt aagttgctgg gggaaaccct gctgcgtctg 420

cctggcgtca atgacacacg gacatacgtt gttatggaag aagtcaagca gagtaatcgt 480

ctggttatta agacgcgcta a 501

<210> 11
<211> 369
<212> PRT
<213> Thermus thermophilus

<400> 11

Met Val Ile Gly Val Pro Lys Glu Ile Lys Thr Leu Glu Asn Arg Val
1 5 10 15

Ala Leu Thr Pro Gly Gly Val Glu Ser Leu Val Arg Arg Gly His Thr
20 25 30

Val Leu Val Glu Arg Gly Ala Gly Glu Gly Ser Gly Leu Ser Asp Ala
35 40 45

Glu Tyr Ala Arg Ala Gly Ala Glu Leu Val Gly Arg Glu Glu Ala Trp
50 55 60

Gly Ala Glu Met Val Val Lys Val Lys Glu Pro Leu Pro Glu Glu Tyr
65 70 75 80

```
Gly Phe Leu Arg Glu Gly Leu Ile Leu Phe Thr Tyr Leu His Leu Ala
                85                  90                  95

Ala Asp Arg Gly Leu Thr Glu Ala Met Leu Arg Ser Gly Val Thr Gly
            100                 105                 110

Ile Ala Tyr Glu Thr Val Gln Leu Pro Asp Gly Thr Leu Pro Leu Leu
        115                 120                 125

Val Pro Met Ser Glu Val Ala Gly Arg Met Ala Pro Gln Val Gly Ala
    130                 135                 140

Gln Phe Leu Glu Lys Pro Lys Gly Gly Arg Gly Val Leu Leu Gly Gly
145                 150                 155                 160

Val Pro Gly Val Ala Pro Ala Ser Val Val Ile Leu Gly Gly Gly Thr
                165                 170                 175

Val Gly Thr Asn Ala Ala Lys Ile Ala Leu Gly Met Gly Ala Gln Val
            180                 185                 190

Thr Ile Leu Asp Val Asn His Lys Arg Leu Gln Tyr Leu Asp Asp Val
        195                 200                 205

Phe Gly Gly Arg Val Ile Thr Leu Thr Ala Thr Glu Ala Asn Ile Lys
    210                 215                 220

Lys Ser Val Gln His Ala Asp Leu Leu Ile Gly Ala Val Leu Val Pro
225                 230                 235                 240

Gly Ala Lys Ala Pro Lys Leu Val Thr Arg Asp Met Leu Ser Leu Met
                245                 250                 255

Lys Glu Gly Ala Val Ile Val Asp Val Ala Val Asp Gln Gly Gly Cys
            260                 265                 270

Val Glu Thr Ile Arg Pro Thr Thr His Ala Glu Pro Thr Tyr Val Val
        275                 280                 285

Asp Gly Val Val His Tyr Gly Val Ala Asn Met Pro Gly Ala Val Pro
    290                 295                 300

Arg Thr Ser Thr Phe Ala Leu Thr Asn Gln Thr Leu Pro Tyr Val Leu
305                 310                 315                 320

Lys Leu Ala Glu Lys Gly Leu Asp Ala Leu Leu Glu Asp Ala Ala Leu
```

325 330 335

Leu Lys Gly Leu Asn Thr His Lys Gly Arg Leu Thr His Pro Gly Val
340 345 350

Ala Glu Ala Phe Gly Leu Pro Tyr Thr Pro Pro Glu Glu Ala Leu Arg
355 360 365

Gly

<210> 12
<211> 1110
<212> DNA
<213> Thermus thermophilus

<400> 12
atggtgatcg gcgtgccgaa ggagatcaag accttggaga accgggtggc cctcacgccc 60

ggcggggtgg agagcctggt caggcgcggc cacaccgtgc tggtggagcg gggggccggg 120

gagggctcgg ggctttccga cgcggagtac gcccgggccg gggccgagct cgtgggccgg 180

gaggaggcct ggggtgcgga gatggtggtg aaggtgaagg agcccctacc cgaggagtac 240

ggcttcctgc gggagggcct catcctcttc acctaccttc acctggccgc ggaccgcggc 300

ctcaccgagg ccatgctccg tagcggggtc acgggcatcg cctacgagac cgtccagctt 360

cccgacggca ccctccccct cctcgtcccc atgagcgagg tggcggggcg gatggccccc 420

caggtggggg cccagttcct ggagaagccc aagggggggcc gggggggtcct cctcgggggg 480

gtgccggggg tggccccggc cagcgtggtg atcctcgggg gcgggaccgt gggcaccaac 540

gcggccaaga tcgccctggg gatgggggcc caggtgacca tcctggacgt gaaccacaag 600

cgcctccagt atctggacga cgtcttcggc gggcgggtga tcaccctcac cgccaccgag 660

gccaacatca aaaagagcgt ccagcacgcg gacctcctca tcggggccgt cctcgtcccc 720

ggggccaagg cccccaagct cgtcacccgg gacatgctct ctctgatgaa ggagggagcg 780

gtgatcgtgg acgtggccgt ggaccagggg gggtgcgtgg agaccatccg gcccaccacc 840

cacgccgagc ccacctacgt ggtggacggg gtggtccact acggggtggc caacatgccc 900

ggggcggtgc ccaggaccag caccttcgcc ctcaccaacc agaccctgcc ctacgtgttg 960

aagctcgcgg agaaggggct ggacgccctt ctggaggacg cggcccttct caaggggctc 1020

aacacccaca aaggccgcct cacccacccc ggggtggccg aggccttcgg cctgccctac 1080

acgcctcccg aggaggcctt gagggggtga 1110

<210> 13
<211> 372
<212> PRT

<213> Lysinibacillus sphaericus

<400> 13

Met Lys Ile Gly Ile Pro Lys Glu Ile Lys Asn Asn Glu Asn Arg Val
1               5                   10                  15

Ala Met Thr Pro Ala Gly Val Val Ser Leu Thr His Ala Gly His Glu
            20                  25                  30

Arg Leu Ala Ile Glu Thr Gly Gly Gly Ile Gly Ser Ser Phe Thr Asp
        35                  40                  45

Ala Glu Tyr Val Ala Ala Gly Ala Ala Tyr Arg Cys Ile Gly Lys Glu
    50                  55                  60

Ala Trp Ala Gln Glu Met Ile Leu Lys Val Lys Glu Pro Val Ala Ser
65                  70                  75                  80

Glu Tyr Asp Tyr Phe Tyr Glu Gly Gln Ile Leu Phe Thr Tyr Leu His
                85                  90                  95

Leu Ala Pro Arg Ala Glu Leu Thr Gln Ala Leu Ile Asp Lys Lys Val
            100                 105                 110

Val Gly Ile Ala Tyr Glu Thr Val Gln Leu Ala Asn Gly Ser Leu Pro
        115                 120                 125

Leu Leu Thr Pro Met Ser Glu Val Ala Gly Lys Met Ala Thr Gln Ile
    130                 135                 140

Gly Ala Gln Tyr Leu Glu Lys Asn His Gly Gly Lys Gly Ile Leu Leu
145                 150                 155                 160

Gly Gly Val Ser Gly Val His Ala Arg Lys Val Thr Val Ile Gly Gly
                165                 170                 175

Gly Ile Ala Gly Thr Asn Ala Ala Lys Ile Ala Val Gly Met Gly Ala
            180                 185                 190

Asp Val Thr Val Ile Asp Leu Ser Pro Glu Arg Leu Arg Gln Leu Glu
        195                 200                 205

Asp Met Phe Gly Arg Asp Val Gln Thr Leu Met Ser Asn Pro Tyr Asn
    210                 215                 220

Ile Ala Glu Ser Val Lys His Ser Asp Leu Val Val Gly Ala Val Leu
225                 230                 235                 240

Ile Pro Gly Ala Lys Ala Pro Lys Leu Val Ser Glu Glu Met Ile Gln
245 250 255

Ser Met Gln Pro Gly Ser Val Val Val Asp Ile Ala Ile Asp Gln Gly
260 265 270

Gly Ile Phe Ala Thr Ser Asp Arg Val Thr Thr His Asp Asp Pro Thr
275 280 285

Tyr Val Lys His Gly Val Val His Tyr Ala Val Ala Asn Met Pro Gly
290 295 300

Ala Val Pro Arg Thr Ser Thr Ile Ala Leu Thr Asn Asn Thr Ile Pro
305 310 315 320

Tyr Ala Leu Gln Ile Ala Asn Lys Gly Tyr Lys Gln Ala Cys Ile Asp
325 330 335

Asn Pro Ala Leu Lys Lys Gly Val Asn Ala Leu Glu Gly His Ile Thr
340 345 350

Tyr Lys Ala Val Ala Glu Ala Gln Gly Leu Pro Tyr Val Asn Val Asp
355 360 365

Glu Leu Ile Gln
370

<210> 14
<211> 1119
<212> DNA
<213> Lysinibacillus sphaericus

<400> 14
atgaagattg gtattccaaa ggaaattaaa aacaacgaaa atcgcgtagc aatgacacca 60

gcaggagttg tatccttaac gcatgctggg cacgagaggt tagctattga aacaggaggt 120

ggtatcggtt caagttttac agatgcagag tacgtagcag caggtgcagc atatcgttgc 180

atcggcaaag aagcgtgggc tcaagaaatg attttaaaag taaaagaacc cgtagcatcg 240

gaatacgatt acttttatga gggacaaatc ttatttacct acttgcactt agcgcccaga 300

gctgaattaa cgcaggcatt aatagataaa aaagttgtag gtattgccta tgaaacggtt 360

caacttgcaa atggttcact acctttatta acaccaatga gtgaagtagc tggtaaaatg 420

gcaacacaaa ttggtgcgca atatttagag aaaaatcacg gtggtaaagg gattttacta 480

ggcggtgtat caggtgtaca cgcgcgtaaa gtaacagtaa ttggtggcgg aatcgcggga 540

acaaacgctg cgaaaattgc agttggtatg ggagcagacg taacagttat tgatttaagt 600

ccagaacgtc tacgtcaatt agaagatatg tttggtcgcg atgttcaaac attaatgtct 660

aacccgtata atattgcaga atctgtgaaa cactcagatt tagttgtcgg tgctgttcta 720

attcctggtg caaaggctcc aaagttagtt tcggaagaaa tgattcaatc gatgcaacca 780

ggttctgttg ttgtggatat tgcgattgac caaggtggaa tttttgcgac atctgatcgt 840

gttacaacac atgatgatcc aacgtatgtt aaacatgggg tagtccatta tgctgttgca 900

aatatgccag ggctgtgcc acgtacttca acgattgctt taacaaataa tacaattcct 960

tatgcgttgc aaattgccaa taaaggctat aagcaagcat gtattgacaa tcctgcattg 1020

aaaaaaggtg tgaatgcatt agaggggcat attacttata aagcggtagc agaagcacaa 1080

ggcttgccat atgtgaatgt ggatgaatta atccaataa 1119

<210> 15
<211> 372
<212> PRT
<213> Geobacillus stearothermophilus

<400> 15

Met Lys Ile Gly Ile Pro Lys Glu Ile Lys Asn Asn Glu Asn Arg Val
1 5 10 15

Ala Ile Thr Pro Ala Gly Val Met Thr Leu Val Lys Ala Gly His Glu
20 25 30

Val Tyr Val Glu Thr Glu Gly Gly Ala Gly Ser Gly Phe Ser Asp Ser
35 40 45

Glu Tyr Glu Lys Ala Gly Ala Ala Asp Arg Cys Arg Thr Trp Arg Asp
50 55 60

Ala Trp Thr Ala Glu Met Val Leu Lys Val Lys Glu Pro Leu Ala Arg
65 70 75 80

Glu Phe Arg Tyr Phe Arg Pro Gly Leu Ile Leu Phe Thr Tyr Leu His
85 90 95

Leu Ala Ala Ala Glu Arg Val Thr Lys Ala Val Val Glu Gln Lys Val
100 105 110

Val Gly Ile Ala Tyr Glu Thr Val Gln Leu Ala Asn Gly Ser Leu Pro
115 120 125

Leu Leu Thr Pro Met Ser Glu Val Ala Gly Arg Met Ser Val Gln Val
130 135 140

Gly Ala Gln Phe Leu Glu Lys Pro His Gly Gly Lys Gly Ile Leu Leu

```
145                 150                 155                 160

Gly Gly Val Pro Gly Val Arg Arg Gly Lys Val Thr Ile Ile Gly Gly
                165                 170                 175

Gly Thr Ala Gly Thr Asn Ala Ala Lys Ile Gly Val Gly Leu Gly Ala
            180                 185                 190

Asp Val Thr Ile Leu Asp Ile Asn Ala Glu Arg Leu Arg Glu Leu Asp
        195                 200                 205

Asp Leu Phe Gly Asp His Val Thr Thr Leu Met Ser Asn Ser Tyr His
    210                 215                 220

Ile Ala Glu Cys Val Arg Glu Ser Asp Leu Val Val Gly Ala Val Leu
225                 230                 235                 240

Ile Pro Gly Ala Lys Ala Lys Leu Val Thr Glu Glu Met Val Arg Ser
                245                 250                 255

Met Thr Pro Gly Ser Val Leu Val Asp Ile Ala Ile Asp Gln Gly Gly
            260                 265                 270

Ile Phe Glu Thr Thr Asp Arg Val Thr Thr His Asp Asp Pro Thr Tyr
        275                 280                 285

Val Lys His Gly Val Val His Tyr Ala Val Ala Asn Met Pro Gly Ala
    290                 295                 300

Val Pro Arg Thr Ser Thr Phe Ala Leu Thr Asn Val Thr Ile Pro Tyr
305                 310                 315                 320

Ala Leu Gln Ile Ala Asn Lys Gly Tyr Arg Ala Gly Cys Leu Asp Asn
                325                 330                 335

Pro Ala Leu Leu Lys Gly Ile Asn Thr Leu Asp Gly His Ile Val Tyr
            340                 345                 350

Glu Ala Val Ala Ala Ala His Asn Met Pro Tyr Thr Asp Val His Ser
        355                 360                 365

Leu Leu His Gly
    370
```

<210>  16
<211>  1119
<212>  DNA
<213>  Geobacillus stearothermophilus

<400> 16
atgaagatcg gcattccaaa agaaatcaaa aacaatgaaa accgcgtcgc catcactccg 60

gcaggcgtga tgacgctcgt caaagcgggg catgaggtgt atgtggagac ggaaggcggc 120

gctgggtcgg ggttttccga ttccgagtat gaaaaagccg gggcagctga tcgttgccga 180

acgtggagag atgcttggac ggcggagatg gtgttgaaag tgaaagagcc gctggctcga 240

gagttccgct attttcgccc cggattgatt ttgtttacgt atttgcattt agccgcggcc 300

gaacgcgtca cgaaagccgt cgtcgagcaa aaagtggtcg gcatcgctta cgagacggtg 360

cagctggcga acggctcgct gccactgttg acgccgatga gtgaagtcgc cggccgcatg 420

tcggtgcaag tcggcgccca gtttctcgag aagccgcacg gggggaaggg cattttgctt 480

ggcggcgtgc ccggagtgcg gcgcggcaaa gtgacgatca tcggcggcgg aacggcgggg 540

acgaacgcgg cgaaaatcgg ggtcggtctc ggtgcagacg tgacgatttt ggacattaac 600

gccgagcggc tgcgcgagct cgatgatttg ttcggcgacc acgtgacgac gctcatgtcc 660

aactcgtacc atatcgccga gtgcgtgcgc gaatcggatt tggtcgtcgg tgccgtcttg 720

atcccggggg cgaaagcgaa gctggtgacg gaagagatgg tgcgctcgat gacgccggga 780

tcggtgttgg tcgacatcgc cattgaccaa ggcggcattt tcgaaacgac cgaccgcgtc 840

acgacgcacg acgatccgac atacgtcaag cacggcgtcg tccattacgc cgtcgccaac 900

atgccgggcg cggtgccgcg cacgtcgaca ttcgcgctta cgaacgtcac gatcccatac 960

gccttgcaaa tcgccaacaa aggctaccgc gccggttgct tggataaccc ggcgctgtta 1020

aaagggatca acacgctcga cgggcacatc gtgtacgaag cggtcgcggc ggcgcacaac 1080

atgccgtata cagatgttca ttcgttgttg cacggatga 1119

<210> 17
<211> 372
<212> PRT
<213> Geobacillus stearothermophilus

<400> 17

Met Lys Ile Gly Ile Pro Lys Glu Ile Lys Asn Asn Glu Asn Arg Val
1 5 10 15

Ala Ile Thr Pro Ala Gly Val Met Thr Leu Val Lys Ala Gly His Asp
20 25 30

Val Tyr Val Glu Thr Glu Ala Gly Ala Gly Ser Gly Phe Ser Asp Ser
35 40 45

Glu Tyr Glu Lys Ala Gly Ala Val Ile Val Thr Lys Ala Glu Asp Ala
50 55 60

```
Trp Ala Ala Glu Met Val Leu Lys Val Lys Glu Pro Leu Ala Glu Glu
65                  70                  75                  80

Phe Arg Tyr Phe Arg Pro Gly Leu Ile Leu Phe Thr Tyr Leu His Leu
                85                  90                  95

Ala Ala Ala Glu Ala Leu Thr Lys Ala Leu Val Glu Gln Lys Val Val
            100                 105                 110

Gly Ile Ala Tyr Glu Thr Val Gln Leu Ala Asn Gly Ser Leu Pro Leu
        115                 120                 125

Leu Thr Pro Met Ser Glu Val Ala Gly Arg Met Ser Val Gln Val Gly
    130                 135                 140

Ala Gln Phe Leu Glu Lys Pro His Gly Gly Lys Gly Ile Leu Leu Gly
145                 150                 155                 160

Gly Val Pro Gly Val Arg Arg Gly Lys Val Thr Ile Ile Gly Gly Gly
                165                 170                 175

Thr Ala Gly Thr Asn Ala Ala Lys Ile Ala Val Gly Leu Gly Ala Asp
            180                 185                 190

Val Thr Ile Leu Asp Ile Asn Ala Glu Arg Leu Arg Glu Leu Asp Asp
        195                 200                 205

Leu Phe Gly Asp Gln Val Thr Thr Leu Met Ser Asn Ser Tyr His Ile
    210                 215                 220

Ala Glu Cys Val Arg Glu Ser Asp Leu Val Val Gly Ala Val Leu Ile
225                 230                 235                 240

Pro Gly Ala Lys Ala Pro Lys Leu Val Thr Glu Glu Met Val Arg Ser
                245                 250                 255

Met Thr Pro Gly Ser Val Leu Val Asp Val Ala Ile Asp Gln Gly Gly
            260                 265                 270

Ile Phe Glu Thr Thr Asp Arg Val Thr Thr His Asp Asp Pro Thr Tyr
        275                 280                 285

Val Lys His Gly Val Val His Tyr Ala Val Ala Asn Met Pro Gly Ala
    290                 295                 300

Val Pro Arg Thr Ser Thr Phe Ala Leu Thr Asn Val Thr Ile Pro Tyr
305                 310                 315                 320
```

Ala Leu Gln Ile Ala Asn Lys Gly Tyr Arg Ala Ala Cys Leu Asp Asn
325 330 335

Pro Ala Leu Leu Lys Gly Ile Asn Thr Leu Asp Gly His Ile Val Tyr
340 345 350

Glu Ala Val Ala Ala Ala His Asn Met Pro Tyr Thr Asp Val His Ser
355 360 365

Leu Leu Gln Gly
370

<210> 18
<211> 1119
<212> DNA
<213> Geobacillus stearothermophilus

<400> 18
atgaagatcg gcattccaaa agaaatcaaa aacaatgaaa accgcgtcgc catcactccg 60

gcaggcgtga tgacgctcgt caaagcgggg catgacgtgt atgtggagac ggaagccggc 120

gctgggtcgg gtttttccga ttccgagtat gaaaaagccg gggcagtgat cgtgacgaaa 180

gcggaagatg cctgggcggc ggagatggtg ttgaaagtga aagaaccgct ggctgaggag 240

ttccgctatt ttcgccccgg attgattttg tttacgtatt tgcatttagc cgcggccgaa 300

gcgctcacga aagcgctcgt cgagcaaaaa gtggtcggca tcgcttacga gacggtgcag 360

cttgcgaacg gctcgctgcc gctgttgacg ccgatgagtg aagtcgccgg ccgcatgtcg 420

gtgcaagtcg gcgcccagtt tctcgagaag ccgcacggcg ggaaaggcat tttgcttggc 480

ggcgtgcccg ggtgcggcg cggcaaagtg acgatcatcg gcggcggcac agcggggacg 540

aacgcggcga aaatcgcggt cggcctcggg gcggacgtga cgattttgga cattaacgcc 600

gagcggctgc gcgagctcga tgatttgttc ggcgaccaag tgacgacgtt gatgtccaac 660

tcgtatcata tcgccgagtg cgtgcgcgaa tccgatttgg tcgtcggcgc cgtcttgatc 720

ccgggggcga aagcgccgaa gcttgtgacg gaagagatgg tgcgctcgat gacgccaggc 780

tcggtgttgg tcgacgtcgc cattgaccaa ggcggcattt ttgaaacgac cgaccgcgtc 840

acgacgcacg acgatccgac atacgtcaag cacggcgtcg tccattacgc cgtcgcgaac 900

atgccgggcg ctgtgccgcg tacgtcaaca ttcgcgctta cgaacgtcac gatcccatac 960

gccttgcaaa tcgccaacaa aggctaccgc gccgcttgcc tcgacaatcc ggcgctgtta 1020

aaagggatca acacgctcga cgggcacatc gtgtacgaag cggtcgcggc ggcgcacaac 1080

atgccgtata cggatgttca ttcgttgctg cagggatga 1119

<210> 19
<211> 372
<212> PRT
<213> Geobacillus stearothermophilus

<400> 19

Met Lys Ile Gly Ile Pro Lys Glu Ile Lys Asn Asn Glu Asn Arg Val
1 5 10 15

Ala Ile Thr Pro Ala Gly Val Met Thr Leu Val Lys Ala Gly His Asp
20 25 30

Val Tyr Val Glu Thr Glu Ala Gly Ala Gly Ser Gly Phe Ser Asp Ser
35 40 45

Glu Tyr Glu Lys Ala Gly Ala Val Ile Val Pro Asn Ala Glu Asp Ala
50 55 60

Trp Thr Ala Glu Met Val Leu Lys Val Lys Glu Pro Leu Ala Glu Glu
65 70 75 80

Phe Arg Tyr Phe Arg Pro Gly Leu Ile Leu Phe Thr Tyr Leu His Leu
85 90 95

Ala Ala Ala Glu Ala Leu Thr Lys Ala Leu Val Glu Gln Lys Val Val
100 105 110

Gly Ile Ala Tyr Glu Thr Val Gln Leu Ala Asn Gly Ser Leu Pro Leu
115 120 125

Leu Thr Pro Met Ser Glu Val Ala Gly Arg Met Ser Val Gln Val Gly
130 135 140

Ala Gln Phe Leu Glu Lys Pro His Gly Gly Lys Gly Ile Leu Leu Gly
145 150 155 160

Gly Val Pro Gly Val Arg Arg Gly Lys Val Thr Ile Ile Gly Gly Gly
165 170 175

Thr Ala Gly Thr Asn Ala Ala Lys Ile Ala Val Gly Leu Gly Ser Asp
180 185 190

Val Thr Ile Leu Asp Ile Asn Ala Glu Arg Leu Arg Glu Leu Asp Asp
195 200 205

Leu Phe Gly Asp His Val Thr Thr Leu Met Ser Asn Ser Tyr His Thr
210 215 220

Ala Glu Cys Val Arg Glu Ser Asp Leu Val Val Gly Ala Val Leu Ile
225 230 235 240

Pro Gly Ala Lys Ala Pro Lys Leu Val Thr Glu Glu Met Val Arg Ser
245 250 255

Met Thr Pro Gly Ser Val Leu Val Asp Ile Ala Ile Asp Gln Gly Gly
260 265 270

Ile Phe Glu Thr Thr Asp Arg Val Thr Thr His Asp Asp Pro Thr Tyr
275 280 285

Val Lys His Gly Val Val His Tyr Ala Val Ala Asn Met Pro Gly Ala
290 295 300

Val Pro Arg Thr Ser Thr Phe Ala Leu Thr Asn Val Thr Ile Pro Tyr
305 310 315 320

Ala Leu Gln Ile Ala Asn Lys Gly Tyr Arg Ala Ala Cys Leu Asp Asn
325 330 335

Pro Ala Leu Leu Lys Gly Ile Asn Thr Leu Asp Gly His Ile Val Tyr
340 345 350

Glu Ala Val Ala Ala Ala His Asn Met Pro Tyr Thr Asp Val His Ser
355 360 365

Leu Leu His Gly
370

<210> 20
<211> 1119
<212> DNA
<213> Geobacillus stearothermophilus

<400> 20
atgaagatcg gcattccaaa agaaatcaaa aacaatgaaa accgcgtcgc catcactccg 60

gcaggcgtga tgacgctcgt caaagcgggg catgacgtgt atgtggagac ggaagccggc 120

gctgggtcgg ggttttccga ttccgagtat gaaaaagccg gggcagtgat cgtgccgaac 180

gcggaagatg cttggacggc ggagatggtg ttgaaagtga aagagccgct ggctgaggag 240

ttccgctatt ttcgccccgg attgattttg tttacgtatt tgcatttagc cgcggccgaa 300

gcgctcacga aagcgctcgt cgagcaaaaa gtggtcggca tcgcttacga gacggtgcag 360

ctggcgaacg gctcgctacc actgttgacg ccgatgagtg aagtcgccgg ccgcatgtcg 420

gtgcaagtcg gcgcccagtt tctcgagaag ccgcacggcg ggaaaggcat tttgcttggc 480

ggcgtgcccg gagtgcggcg cggcaaagtg acgatcatcg gcggcggaac ggcggggacg 540

aacgcggcga aaatcgcggt cggtctcggg tcagacgtga cgattttgga cattaacgcc 600

gagcggctgc gcgagctcga tgatttgttc ggcgaccacg tgacgacgct catgtccaac 660

tcgtaccata ccgccgagtg cgtgcgcgaa tcggatttgg tcgtcggtgc cgtcttgatc 720

ccgggggcga aagcgccgaa gctggtgacg gaagagatgg tgcgctcgat gacgccggga 780

tcggtgttgg tcgacatcgc cattgaccaa ggcggcattt tcgaaacgac cgaccgcgtc 840

acgacgcacg acgatccgac atacgtcaag cacggcgtcg tccattacgc cgtcgccaac 900

atgccgggcg cggtgccgcg cacgtcgaca ttcgcgctta cgaacgtcac gatcccatac 960

gccttgcaaa tcgccaacaa aggctaccgc gccgcgtgct tggataaccc ggcgctgtta 1020

aaggggatca acacgctcga cgggcacatc gtgtacgaag cggtcgcggc ggcgcacaac 1080

atgccgtata cagatgttca ttcgttgttg cacggatga 1119

<210> 21
<211> 371
<212> PRT
<213> Mycobacterium tuberculosis

<400> 21

Met Arg Val Gly Ile Pro Thr Glu Thr Lys Asn Asn Glu Phe Arg Val
1 5 10 15

Ala Ile Thr Pro Ala Gly Val Ala Glu Leu Thr Arg Arg Gly His Glu
20 25 30

Val Leu Ile Gln Ala Gly Ala Gly Glu Gly Ser Ala Ile Thr Asp Ala
35 40 45

Asp Phe Lys Ala Ala Gly Ala Gln Leu Val Gly Thr Ala Asp Gln Val
50 55 60

Trp Ala Asp Ala Asp Leu Leu Leu Lys Val Lys Glu Pro Ile Ala Ala
65 70 75 80

Glu Tyr Gly Arg Leu Arg His Gly Gln Ile Leu Phe Thr Phe Leu His
85 90 95

Leu Ala Ala Ser Arg Ala Cys Thr Asp Ala Leu Leu Asp Ser Gly Thr
100 105 110

Thr Ser Ile Ala Tyr Glu Thr Val Gln Thr Ala Asp Gly Ala Leu Pro
115 120 125

Leu Leu Ala Pro Met Ser Glu Val Ala Gly Arg Leu Ala Ala Gln Val
130 135 140

```
Gly Ala Tyr His Leu Met Arg Thr Gln Gly Gly Arg Gly Val Leu Met
145                 150                 155                 160

Gly Gly Val Pro Gly Val Glu Pro Ala Asp Val Val Val Ile Gly Ala
                165                 170                 175

Gly Thr Ala Gly Tyr Asn Ala Ala Arg Ile Ala Asn Gly Met Gly Ala
            180                 185                 190

Thr Val Thr Val Leu Asp Ile Asn Ile Asp Lys Leu Arg Gln Leu Asp
        195                 200                 205

Ala Glu Phe Cys Gly Arg Ile His Thr Arg Tyr Ser Ser Ala Tyr Glu
    210                 215                 220

Leu Glu Gly Ala Val Lys Arg Ala Asp Leu Val Ile Gly Ala Val Leu
225                 230                 235                 240

Val Pro Gly Ala Lys Ala Pro Lys Leu Val Ser Asn Ser Leu Val Ala
                245                 250                 255

His Met Lys Pro Gly Ala Val Leu Val Asp Ile Ala Ile Asp Gln Gly
            260                 265                 270

Gly Cys Phe Glu Gly Ser Arg Pro Thr Thr Tyr Asp His Pro Thr Phe
        275                 280                 285

Ala Val His Asp Thr Leu Phe Tyr Cys Val Ala Asn Met Pro Ala Ser
    290                 295                 300

Val Pro Lys Thr Ser Thr Tyr Ala Leu Thr Asn Ala Thr Met Pro Tyr
305                 310                 315                 320

Val Leu Glu Leu Ala Asp His Gly Trp Arg Ala Ala Cys Arg Ser Asn
                325                 330                 335

Pro Ala Leu Ala Lys Gly Leu Ser Thr His Glu Gly Ala Leu Leu Ser
            340                 345                 350

Glu Arg Val Ala Thr Asp Leu Gly Val Pro Phe Thr Glu Pro Ala Ser
        355                 360                 365

Val Leu Ala
    370
```

<210> 22

<211> 1122
<212> DNA
<213> Mycobacterium tuberculosis

<400> 22
atgcgcgtcg gtattccgac cgagaccaaa aacaacgaat tccaattccg ggtggccatc 60

accccggccg gcgtcgcgga actaacccgt cgtggccatg aggtgctcat ccaggcaggt 120

gccggagagg gctcggctat caccgacgcg gatttcaagg cggcaggcgc gcaactggtc 180

ggcaccgccg accaggtgtg ggccgacgct gatttattgc tcaaggtcaa agaaccgata 240

gcggcggaat acggccgcct gcgacacggg cagatcttgt tcacgttctt gcatttggcc 300

gcgtcacgtg cttgcaccga tgcgttgttg gattccggca ccacgtcaat tgcctacgag 360

accgtccaga ccgccgacgg cgcactaccc ctgcttgccc cgatgagcga agtcgccggt 420

cgactcgccg cccaggttgg cgcttaccac ctgatgcgaa cccaaggggg ccgcggtgtg 480

ctgatgggcg gggtgcccgg cgtcgaaccg gccgacgtcg tggtgatcgg cgccggcacc 540

gccggctaca acgcagcccg catcgccaac ggcatgggcg cgaccgttac ggttctagac 600

atcaacatcg acaaacttcg gcaactcgac gccgagttct gcggccggat ccacactcgc 660

tactcatcgg cctacgagct cgagggtgcc gtcaaacgtg ccgacctggt gattggggcc 720

gtcctggtgc caggcgccaa ggcacccaaa ttagtctcga attcacttgt cgcgcatatg 780

aaaccaggtg cggtactggt ggatatagcc atcgaccagg gcggctgttt cgaaggctca 840

cgaccgacca cctacgacca cccgacgttc gccgtgcacg acacgctgtt ttactgcgtg 900

gcgaacatgc ccgcctcggt gccgaagacg tcgacctacg cgctgaccaa cgcgacgatg 960

ccgtatgtgc tcgagcttgc cgaccatggc tggcgggcgg cgtgccggtc gaatccggca 1020

ctagccaaag gtctttcgac gcacgaaggg gcgttactgt ccgaacgggt ggccaccgac 1080

ctgggggtgc cgttcaccga gcccgccagc gtgctggcct ga 1122

<210> 23
<211> 322
<212> PRT
<213> Archaeoglobus fulgidus

<400> 23

Met Glu Thr Leu Ile Leu Thr Gln Glu Glu Val Glu Ser Leu Ile Ser
1 5 10 15

Met Asp Glu Ala Met Asn Ala Val Glu Glu Ala Phe Arg Leu Tyr Ala
20 25 30

Leu Gly Lys Ala Gln Met Pro Pro Lys Val Tyr Leu Glu Phe Glu Lys
35 40 45

Gly Asp Leu Arg Ala Met Pro Ala His Leu Met Gly Tyr Ala Gly Leu
    50                  55                  60

Lys Trp Val Asn Ser His Pro Gly Asn Pro Asp Lys Gly Leu Pro Thr
65                  70                  75                  80

Val Met Ala Leu Met Ile Leu Asn Ser Pro Glu Thr Gly Phe Pro Leu
                85                  90                  95

Ala Val Met Asp Ala Thr Tyr Thr Thr Ser Leu Arg Thr Gly Ala Ala
            100                 105                 110

Gly Gly Ile Ala Ala Lys Tyr Leu Ala Arg Lys Asn Ser Ser Val Phe
        115                 120                 125

Gly Phe Ile Gly Cys Gly Thr Gln Ala Tyr Phe Gln Leu Glu Ala Leu
    130                 135                 140

Arg Arg Val Phe Asp Ile Gly Glu Val Lys Ala Tyr Asp Val Arg Glu
145                 150                 155                 160

Lys Ala Ala Lys Lys Phe Val Ser Tyr Cys Glu Asp Arg Gly Ile Ser
                165                 170                 175

Ala Ser Val Gln Pro Ala Glu Glu Ala Ser Arg Cys Asp Val Leu Val
            180                 185                 190

Thr Thr Thr Pro Ser Arg Lys Pro Val Val Lys Ala Glu Trp Val Glu
        195                 200                 205

Glu Gly Thr His Ile Asn Ala Ile Gly Ala Asp Gly Pro Gly Lys Gln
    210                 215                 220

Glu Leu Asp Val Glu Ile Leu Lys Lys Ala Lys Ile Val Val Asp Asp
225                 230                 235                 240

Leu Glu Gln Ala Lys His Gly Gly Glu Ile Asn Val Ala Val Ser Lys
                245                 250                 255

Gly Val Ile Gly Val Glu Asp Val His Ala Thr Ile Gly Glu Val Ile
            260                 265                 270

Ala Gly Leu Lys Asp Gly Arg Glu Ser Asp Glu Glu Ile Thr Ile Phe
        275                 280                 285

Asp Ser Thr Gly Leu Ala Ile Gln Asp Val Ala Val Ala Lys Val Val
    290                 295                 300

Tyr Glu Asn Ala Leu Ser Lys Asn Val Gly Ser Lys Ile Lys Phe Phe
305 310 315 320

Arg Ile

<210> 24
<211> 969
<212> DNA
<213> Archaeoglobus fulgidus

<400> 24
atggagactc ttattttgac tcaggaggaa gtagaatccc tgatatcgat ggacgaggcg 60

atgaatgctg ttgaggaggc gttcagactt tacgctcttg gtaaagctca aatgccgcca 120

aaggtgtatc ttgagttcga aaaaggcgat ttaagggcta tgccggcgca tttaatgggg 180

tacgcaggat tgaagtgggt gaactcccat cccggaaatc cggataaagg cctgccaaca 240

gtcatggcgt tgatgatact gaacagccct gagacggggt tcccttggc tgtgatggac 300

gcaacctaca cgacctctct gagaaccggt gctgctggag gaatagcggc gaagtatctg 360

gcaaggaaga acagcagcgt ttttggattt atcggctgcg gaactcaggc gtatttccag 420

ctcgaagctc tgaggagagt gtttgatata ggagaagtca aggcctacga tgttagggaa 480

aaggcagcga aaaagttcgt tagctattgc gaagataggg gaatctcagc ttccgtgcag 540

cctgctgagg aggcgagcag atgcgatgtc cttgtcacca caactccgtc acgaaaacca 600

gttgttaagg ctgagtgggt tgaggagggg acgcacataa acgcaatcgg tgcggacggg 660

cctggaaagc aggaactgga tgtagaaata cttaaaaagg ctaaaattgt tgtcgatgac 720

cttgaacagg ccaagcacgg cggggagatt aatgtggcgg tttcaaaagg cgtcataggt 780

gttgaagatg ttcacgcaac catcggggag gttatagccg gtctgaagga tggaagggag 840

agcgatgagg agattacaat cttcgactcc acaggcttgg cgattcagga cgtggctgtt 900

gcaaaagtcg tttatgagaa cgcccttagc aagaacgtgg ggagtaaaat aaagtttttc 960

aggatatga 969

<210> 25
<211> 378
<212> PRT
<213> Bacillus subtilis

<400> 25

Met Ile Ile Gly Val Pro Lys Glu Ile Lys Asn Asn Glu Asn Arg Val
1 5 10 15

Ala Leu Thr Pro Gly Gly Val Ser Gln Leu Ile Ser Asn Gly His Arg
20 25 30

Val Leu Val Glu Thr Gly Ala Gly Leu Gly Ser Gly Phe Glu Asn Glu
35 40 45

Ala Tyr Glu Ser Ala Gly Ala Glu Ile Ile Ala Asp Pro Lys Gln Val
50 55 60

Trp Asp Ala Glu Met Val Met Lys Val Lys Glu Pro Leu Pro Glu Glu
65 70 75 80

Tyr Val Tyr Phe Arg Lys Gly Leu Val Leu Phe Thr Tyr Leu His Leu
85 90 95

Ala Ala Glu Pro Glu Leu Ala Gln Ala Leu Lys Asp Lys Gly Val Thr
100 105 110

Ala Ile Ala Tyr Glu Thr Val Ser Glu Gly Arg Thr Leu Pro Leu Leu
115 120 125

Thr Pro Met Ser Glu Val Ala Gly Arg Met Ala Ala Gln Ile Gly Ala
130 135 140

Gln Phe Leu Glu Lys Pro Lys Gly Gly Lys Gly Ile Leu Leu Ala Gly
145 150 155 160

Val Pro Gly Val Ser Arg Gly Lys Val Thr Ile Ile Gly Gly Gly Val
165 170 175

Val Gly Thr Asn Ala Ala Lys Met Ala Val Gly Leu Gly Ala Asp Val
180 185 190

Thr Ile Ile Asp Leu Asn Ala Asp Arg Leu Arg Gln Leu Asp Asp Ile
195 200 205

Phe Gly His Gln Ile Lys Thr Leu Ile Ser Asn Pro Val Asn Ile Ala
210 215 220

Asp Ala Val Ala Glu Ala Asp Leu Leu Ile Cys Ala Val Leu Ile Pro
225 230 235 240

Gly Ala Lys Ala Pro Thr Leu Val Thr Glu Glu Met Val Lys Gln Met
245 250 255

Lys Pro Gly Ser Val Ile Val Asp Val Ala Ile Asp Gln Gly Gly Ile
260 265 270

Val Glu Thr Val Asp His Ile Thr Thr His Asp Gln Pro Thr Tyr Glu

275 280 285

Lys His Gly Val Val His Tyr Ala Val Ala Asn Met Pro Gly Ala Val
290 295 300

Pro Arg Thr Ser Thr Ile Ala Leu Thr Asn Val Thr Val Pro Tyr Ala
305 310 315 320

Leu Gln Ile Ala Asn Lys Gly Ala Val Lys Ala Leu Ala Asp Asn Thr
325 330 335

Ala Leu Arg Ala Gly Leu Asn Thr Ala Asn Gly His Val Thr Tyr Glu
340 345 350

Ala Val Ala Arg Asp Leu Gly Tyr Glu Tyr Val Pro Ala Glu Lys Ala
355 360 365

Leu Gln Asp Glu Ser Ser Val Ala Gly Ala
370 375

<210> 26
<211> 1137
<212> DNA
<213> Bacillus subtilis

<400> 26
atgatcatag gggttcctaa agagataaaa aacaatgaaa accgtgtcgc attaacaccc 60

gggggcgttt ctcagctcat ttcaaacggc caccgggtgc tggttgaaac aggcgcgggc 120

cttggaagcg gatttgaaaa tgaagcctat gagtcagcag gagcggaaat cattgctgat 180

ccgaagcagg tctgggacgc cgaaatggtc atgaaagtaa aagaaccgct gccggaagaa 240

tatgtttatt ttcgcaaagg acttgtgctg tttacgtacc ttcatttagc agctgagcct 300

gagcttgcac aggccttgaa ggataaagga gtaactgcca tcgcatatga aacggtcagt 360

gaaggccgga cattgcctct tctgacgcca atgtcagagg ttgcgggcag aatggcagcg 420

caaatcggcg ctcaattctt agaaaagcct aaaggcggaa aaggcattct gcttgccggg 480

gtgcctggcg tttcccgcgg aaaagtaaca attatcggag gaggcgttgt cgggacaaac 540

gcggcgaaaa tggctgtcgg cctcggtgca gatgtgacga tcattgactt aaacgcagac 600

cgcttgcgcc agcttgatga catcttcggc catcagatta aaacgttaat ttctaatccg 660

gtcaatattg ctgatgctgt ggcggaagcg gatctcctca tttgcgcggt attaattccg 720

ggtgctaaag ctccgactct tgtcactgag gaaatggtaa aacaaatgaa acccggttca 780

gttattgttg atgtagcgat cgaccaaggc ggcatcgtcg aaactgtcga ccatatcaca 840

acacatgatc agccaacata tgaaaaacac ggggttgtgc attatgctgt agcgaacatg 900

```
ccaggcgcag tccctcgtac atcaacaatc gccctgacta acgttactgt tccatacgcg      960

ctgcaaatcg cgaacaaagg ggcagtaaaa gcgctcgcag acaatacggc actgagagcg     1020

ggtttaaaca ccgcaaacgg acacgtgacc tatgaagctg tagcaagaga tctaggctat     1080

gagtatgttc ctgccgagaa agctttacag gatgaatcat ctgtggcggg tgcttaa        1137


<210>  27
<211>  377
<212>  PRT
<213>  Klebsiella aerogenes

<400>  27

Met Ile Ile Gly Val Pro Lys Glu Ile Lys Asn Asn Glu Asn Arg Val
1               5                   10                  15


Ala Met Thr Pro Ala Gly Val Val His Leu Leu Asn Ala Gly His Lys
            20                  25                  30


Val Ile Ile Glu Thr Asn Ala Gly Leu Gly Ser Gly Phe Thr Asn Glu
        35                  40                  45


Glu Tyr Lys Gln Ala Gly Ala Glu Ile Ile Glu Ser Ala Ser Asp Val
    50                  55                  60


Trp Thr Lys Ala Asp Met Ile Met Lys Val Lys Glu Pro Leu Ala Ser
65                  70                  75                  80


Glu Tyr Gly Tyr Phe Arg Lys Gly Leu Ile Leu Phe Thr Tyr Leu His
                85                  90                  95


Leu Ala Ala Glu Pro Glu Leu Thr Lys Ala Leu Val Asp Ser Glu Val
            100                 105                 110


Ile Ala Ile Ala Tyr Glu Thr Val Thr Val Asn Arg Thr Leu Pro Leu
        115                 120                 125


Leu Ser Pro Met Ser Glu Val Ala Gly Arg Met Ala Ala Gln Val Gly
    130                 135                 140


Ala Gln Phe Leu Glu Lys Thr Gln Gly Gly Lys Gly Ile Leu Leu Ser
145                 150                 155                 160


Gly Val Pro Gly Val Lys Arg Gly Lys Val Thr Ile Ile Gly Gly Gly
                165                 170                 175


Met Val Gly Thr Asn Ala Ala Lys Ile Ala Val Gly Leu Gly Ala Asp
            180                 185                 190
```

Val Thr Ile Ile Asp Leu Asn Pro Asp Arg Leu Arg Gln Leu Glu Asp
        195                 200                 205

Ile Phe Gly Thr Ser Val Gln Thr Leu Met Ser Asn Pro Tyr Asn Ile
    210                 215                 220

Ala Glu Ala Val Lys Glu Ser Asp Leu Val Ile Gly Ser Val Leu Ile
225                 230                 235                 240

Pro Gly Ala Lys Ala Pro Lys Leu Val Thr Glu Glu Met Val Lys Ser
                245                 250                 255

Met Gln Pro Gly Ser Val Ile Val Asp Val Ala Ile Asp Gln Gly Gly
            260                 265                 270

Asn Phe Glu Thr Val Asp His Ile Thr Thr His Asp Asp Pro Thr Tyr
        275                 280                 285

Val Lys His Gly Val Val His Tyr Ala Val Ala Asn Met Pro Gly Ala
    290                 295                 300

Val Pro Arg Thr Ala Thr Ile Ala Leu Thr Asn Val Thr Ile Pro Tyr
305                 310                 315                 320

Ala Val Gln Ile Ala Thr Lys Gly Val Val Lys Ala Val Asn Asp Asn
                325                 330                 335

Pro Ala Ile Lys Ala Gly Val Asn Val Ala Asn Gly His Val Thr Phe
            340                 345                 350

Glu Ala Val Ala Asn Asp Leu Gly Tyr Lys Tyr Val Thr Val Glu Glu
        355                 360                 365

Ala Ile Ser Lys Glu Ala Ile Asn Ala
    370                 375

<210> 28
<211> 1134
<212> DNA
<213> Klebsiella aerogenes

<400> 28
atgattatcg gtgttcctaa ggaaattaaa aataatgaaa atcgtgtggc aatgactcct 60

gctggtgtcg ttcatttatt aaatgctgga cataaagtta ttattgaaac aaacgctgga 120

ttaggcagtg gttttacgaa cgaagaatat aaacaagctg gtgctgaaat tattgaaagc 180

gcttcagatg tatggacgaa agctgatatg attatgaagg ttaaagaacc gcttgcatct 240

```
gagtacggtt atttccgtaa aggattaatc ttattcacat atcttcactt agctgcagaa      300

cctgaattaa caaaagcgtt ggtagatagt gaagtgattg ccattgcata tgaaacagtt      360

actgtaaatc gtacattacc acttctttca ccaatgagtg aagtagcagg cagaatggca      420

gcacaagtcg gagctcaatt ccttgaaaag acacaaggtg gtaaaggaat tctactaagt      480

ggtgtacctg gagtaaaacg tggcaaagta acgattattg gtggcggaat ggttggaaca      540

aacgctgcaa aaatcgcagt cggtctagga gcggatgtaa caatcatcga tttaaatcca      600

gatcgtttac gtcaattaga agacattttt ggtacaagtg ttcaaacatt aatgtcaaat      660

ccatacaata ttgctgaagc tgttaaagaa tccgatttag ttattggatc agtactcatt      720

cctggtgcaa aagctccgaa attagtgaca gaagaaatgg ttaaatccat gcaaccagga      780

tctgttattg tagacgttgc aatcgaccaa ggtggaaact tcgaaacagt tgatcatatt      840

acaactcatg atgacccaac ttatgttaaa cacggtgttg ttcattatgc agtagctaat      900

atgcctggtg cagttccacg tacagctaca attgccttaa caaatgtaac gattccttat      960

gctgtacaaa ttgcgacaaa aggtgtagtt aaagcagtga atgataatcc tgcaattaaa     1020

gctggtgtga acgtagcgaa tggccatgta acatttgaag cagtagcaaa cgatcttggc     1080

tacaaatatg taacagtaga agaagcaatt tctaaagaag caatcaatgc ataa           1134


<210>  29
<211>  361
<212>  PRT
<213>  Phormidium lapideum

<400>  29

Met Glu Ile Gly Val Pro Lys Glu Ile Lys Asn Gln Glu Phe Arg Val
1               5                   10                  15


Gly Leu Ser Pro Ser Ser Val Arg Thr Leu Val Glu Ala Gly His Thr
            20                  25                  30


Val Phe Ile Glu Thr Gln Ala Gly Ile Gly Ala Gly Phe Ala Asp Gln
        35                  40                  45


Asp Tyr Val Gln Ala Gly Ala Gln Val Val Pro Ser Ala Lys Asp Ala
    50                  55                  60


Trp Ser Arg Glu Met Val Val Lys Val Lys Glu Pro Leu Pro Ala Glu
65                  70                  75                  80


Tyr Asp Leu Met Gln Lys Asp Gln Leu Leu Phe Thr Tyr Leu His Leu
                85                  90                  95


Ala Ala Ala Arg Glu Leu Thr Glu Gln Leu Met Arg Val Gly Leu Thr
```

100 105 110

Ala Ile Ala Tyr Glu Thr Val Glu Leu Pro Asn Arg Ser Leu Pro Leu
115 120 125

Leu Thr Pro Met Ser Ile Ile Ala Gly Arg Leu Ser Val Gln Phe Gly
130 135 140

Ala Arg Phe Leu Glu Arg Gln Gln Gly Gly Arg Gly Val Leu Leu Gly
145 150 155 160

Gly Val Pro Gly Val Lys Pro Gly Lys Val Val Ile Leu Gly Gly Gly
165 170 175

Val Val Gly Thr Glu Ala Ala Lys Met Ala Val Gly Leu Gly Ala Gln
180 185 190

Val Gln Ile Phe Asp Ile Asn Val Glu Arg Leu Ser Tyr Leu Glu Thr
195 200 205

Leu Phe Gly Ser Arg Val Glu Leu Leu Tyr Ser Asn Ser Ala Glu Ile
210 215 220

Glu Thr Ala Val Ala Glu Ala Asp Leu Leu Ile Gly Ala Val Leu Val
225 230 235 240

Pro Gly Arg Arg Ala Pro Ile Leu Val Pro Ala Ser Leu Val Glu Gln
245 250 255

Met Arg Thr Gly Ser Val Ile Val Asp Val Ala Val Asp Gln Gly Gly
260 265 270

Cys Val Glu Thr Leu His Pro Thr Ser His Thr Gln Pro Thr Tyr Glu
275 280 285

Val Phe Gly Val Val His Tyr Gly Val Pro Asn Met Pro Gly Ala Val
290 295 300

Pro Trp Thr Ala Thr Gln Ala Leu Asn Asn Ser Thr Leu Pro Tyr Val
305 310 315 320

Val Lys Leu Ala Asn Gln Gly Leu Lys Ala Leu Glu Thr Asp Asp Ala
325 330 335

Leu Ala Lys Gly Leu Asn Val Gln Ala His Arg Leu Val His Pro Ala
340 345 350

Val Gln Gln Val Phe Pro Asp Leu Ala
355 360

<210> 30
<211> 1086
<212> DNA
<213> Phormidium lapideum

<400> 30
atggaaatcg gcgttcccaa agagattaag aatcaggaat ttcgggtggg gctgagtccc 60

agcagcgtcc gcacgctggt cgaagcagga cacacagtat ttatcgaaac ccaggcgggc 120

atcggcgcag gatttgccga tcaggactat gttcaagctg gcgctcaggt cgtgccttct 180

gcaaaggatg cctggagccg agagatggtg gtgaaggtca aggaaccgct gcctgcggaa 240

tatgacctga tgcaaaaaga tcaattgctg tttacctacc tgcacctggc ggcggcaaga 300

gaactgacgg agcaactgat gcgggtgggg ctgacggcga tcgcctatga aaccgtcgaa 360

ctgcccaacc gcagcctgcc cctgctgact cccatgagca tcatcgctgg gcggctctcg 420

gttcagtttg ggcccgcttt tttggaacga cagcagggtg ggcgcggcgt gctgctaggc 480

ggcgtgccgg gggtaaaacc aggcaaagtc gtgatcttgg gcggcggtgt ggtgggcacc 540

gaagctgcca agatggccgt cgggctgggc gctcaggtgc aaatttttga catcaatgtg 600

gagcggctgt cttacctgga aaccctgttt ggctcgcggg tcgagctgct ctacagcaat 660

tccgccgaga tcgagactgc ggtcgccgag gctgatttgc tcatcggcgc agtgctggta 720

ccgggccgac gtgcgcccat cctagtgcct gccagcctgg tggagcaaat gcggacaggc 780

tcggtgatcg tagacgtggc ggtggatcag ggcggctgcg tagagacgct gcacccaact 840

tcccacacgc agcccaccta cgaggtgttt ggcgtggttc actatggcgt gccgaatatg 900

cccggagccg ttccctggac agcgacgcag gcactcaaca acagcaccct gccctacgtg 960

gtgaagctgg ccaaccaggg acttaaggca ctggaaaccg atgacgcgct agccaagggg 1020

ctaaacgtgc aggcccatcg cttggtgcat cccgcagtgc agcaggtttt ccccgatctg 1080

gcgtag 1086

<210> 31
<211> 293
<212> PRT
<213> Escherichia coli

<400> 31

Met Thr Arg Gln Lys Ala Thr Leu Ile Gly Leu Ile Ala Ile Val Leu
1 5 10 15

Trp Ser Thr Met Val Gly Leu Ile Arg Gly Val Ser Glu Gly Leu Gly
20 25 30

Pro Val Gly Gly Ala Ala Ala Ile Tyr Ser Leu Ser Gly Leu Leu Leu
35 40 45

Ile Phe Thr Val Gly Phe Pro Arg Ile Arg Gln Ile Pro Lys Gly Tyr
50 55 60

Leu Leu Ala Gly Ser Leu Leu Phe Val Ser Tyr Glu Ile Cys Leu Ala
65 70 75 80

Leu Ser Leu Gly Tyr Ala Ala Thr His His Gln Ala Ile Glu Val Gly
85 90 95

Met Val Asn Tyr Leu Trp Pro Ser Leu Thr Ile Leu Phe Ala Ile Leu
100 105 110

Phe Asn Gly Gln Lys Thr Asn Trp Leu Ile Val Pro Gly Leu Leu Leu
115 120 125

Ala Leu Val Gly Val Cys Trp Val Leu Gly Gly Asp Asn Gly Leu His
130 135 140

Tyr Asp Glu Ile Ile Asn Asn Ile Thr Thr Ser Pro Leu Ser Tyr Phe
145 150 155 160

Leu Ala Phe Ile Gly Ala Phe Ile Trp Ala Ala Tyr Cys Thr Val Thr
165 170 175

Asn Lys Tyr Ala Arg Gly Phe Asn Gly Ile Thr Val Phe Val Leu Leu
180 185 190

Thr Gly Ala Ser Leu Trp Val Tyr Tyr Phe Leu Thr Pro Gln Pro Glu
195 200 205

Met Ile Phe Ser Thr Pro Val Met Ile Lys Leu Ile Ser Ala Ala Phe
210 215 220

Thr Leu Gly Phe Ala Tyr Ala Ala Trp Asn Val Gly Ile Leu His Gly
225 230 235 240

Asn Val Thr Ile Met Ala Val Gly Ser Tyr Phe Thr Pro Val Leu Ser
245 250 255

Ser Ala Leu Ala Ala Val Leu Leu Ser Ala Pro Leu Ser Phe Ser Phe
260 265 270

Trp Gln Gly Ala Leu Met Val Cys Gly Gly Ser Leu Leu Cys Trp Leu
275 280 285

Ala Thr Arg Arg Gly
290

<210> 32
<211> 882
<212> DNA
<213> Escherichia coli

<400> 32
atgacacgac aaaaagcaac gctcataggg ctgatagcga tcgtcctgtg gagcacgatg 60

gtaggattga ttcgcggtgt cagtgagggg ctcggcccgg tcggcggcgc agctgctatc 120

tattcattaa gcgggctgct gttaatcttc acggttggat ttccgcgtat tcggcaaatc 180

ccgaaaggct atttactcgc cgggagtctg ttattcgtca gctatgaaat ctgtctggcg 240

ctttccttag ggtatgcggc gacccatcat caggcgattg aagtgggtat ggtgaactat 300

ctgtggccca gcctgacaat tctctttgcc attctgttta atggtcagaa aaccaactgg 360

ttgattgtac ctggattatt attagccctc gtcggcgtct gttgggtgtt aggcggtgac 420

aatgggttac attatgatga aatcatcaat aatatcacca ccagcccatt gagttatttc 480

ctggcgttca ttggtgcgtt tatctgggca gcctattgca cagtaacgaa taaatacgca 540

cgcggattta atggaattac cgtttttgtc ctgctaacgg gagcaagtct gtgggtttac 600

tattttctta cgccacaacc agaaatgata tttagcacgc ccgtcatgat taaactcatc 660

tctgcggcat ttaccttagg atttgcttat gctgcatgga atgtcggtat attgcatggc 720

aatgtcacca ttatggcggt aggttcgtat tttacgcctg tactttcctc agcgcttgca 780

gccgtgctgc tcagcgcccc gctgtcgttc tcgttctggc aaggcgcgct gatggtctgc 840

ggcggttccc tgctctgctg gctggcgaca cgtcgtggtt aa 882

<210> 33
<211> 149
<212> PRT
<213> Escherichia coli

<400> 33

Met Phe Ser Pro Gln Ser Arg Leu Arg His Ala Val Ala Asp Thr Phe
1 5 10 15

Ala Met Val Val Tyr Cys Ser Val Val Asn Met Cys Ile Glu Val Phe
20 25 30

Leu Ser Gly Met Ser Phe Glu Gln Ser Phe Tyr Ser Arg Leu Val Ala
35 40 45

Ile Pro Val Asn Ile Leu Ile Ala Trp Pro Tyr Gly Met Tyr Arg Asp

50 55 60

Leu Phe Met Arg Ala Ala Arg Lys Val Ser Pro Ser Gly Trp Ile Lys
65 70 75 80

Asn Leu Ala Asp Ile Leu Ala Tyr Val Thr Phe Gln Ser Pro Val Tyr
85 90 95

Val Ala Ile Leu Leu Val Val Gly Ala Asp Trp His Gln Ile Met Ala
100 105 110

Ala Val Ser Ser Asn Ile Val Val Ser Met Leu Met Gly Ala Val Tyr
115 120 125

Gly Tyr Phe Leu Asp Tyr Cys Arg Arg Leu Phe Lys Val Ser Arg Tyr
130 135 140

Gln Gln Val Lys Ala
145

<210> 34
<211> 450
<212> DNA
<213> Escherichia coli

<400> 34
atgttctcac cgcagtcacg cttgcgtcat gcagttgcag atacgttcgc gatggttgtt 60

tactgttctg tcgtgaacat gtgtattgaa gttttcctct ccggaatgag cttcgaacag 120

tctttttatt ccagattggt agcgattccg gtgaacatct taattgcatg gccatacggt 180

atgtaccgtg atctgtttat gcgcgcggca cgcaaagtta gcccgtcggg ctggataaaa 240

aatctggcgg atatcctggc ttatgtgacg ttccagtcac cggtgtatgt ggcgatcttg 300

ttagtggtgg gcgcagactg gcatcagatt atggcggcgg tcagttcaaa catcgttgtt 360

tcgatgttga tgggggcggt ttatggctac ttcctcgatt attgccgccg actgtttaaa 420

gtcagccgtt accagcaggt aaaagcctga 450

<210> 35
<211> 1203
<212> DNA
<213> Escherichia coli

<400> 35
atgtcgagta agttagtact ggttctgaac tgcggtagtt cttcactgaa atttgccatc 60

atcgatgcag taaatggtga agagtacctt tctggtttag ccgaatgttt ccacctgccc 120

gaagcacgta tcaaatggaa aatggacggc aataaacagg aagcggcttt aggtgcaggc 180

gccgctcaca gcgaagcgct caactttatc gttaatacta ttctggcaca aaaaccagaa 240

ctgtctgcgc agctgactgc tatcggtcac cgtatcgtac acggcggcga aaagtatacc 300

agctccgtag tgatcgatga gtctgttatt cagggtatca aagatgcagc ttcttttgca 360

ccgctgcaca acccggctca cctgatcggt atcgaagaag ctctgaaatc tttcccacag 420

ctgaaagaca aaaacgttgc tgtatttgac accgcgttcc accagactat gccggaagag 480

tcttacctct acgccctgcc ttacaacctg tacaaagagc acggcatccg tcgttacggc 540

gcgcacggca ccagccactt ctatgtaacc caggaagcgg caaaaatgct gaacaaaccg 600

gtagaagaac tgaacatcat cacctgccac ctgggcaacg gtggttccgt ttctgctatc 660

cgcaacggta aatgcgttga cacctctatg ggcctgaccc cgctggaagg tctggtcatg 720

ggtacccgtt ctggtgatat cgatccggcg atcatcttcc acctgcacga caccctgggc 780

atgagcgttg acgcaatcaa caaactgctg accaaagagt ctggcctgct gggtctgacc 840

gaagtgacca gcgactgccg ctatgttgaa gacaactacg cgacgaaaga agacgcgaag 900

cgcgcaatgg acgtttactg ccaccgcctg gcgaaataca tcggtgccta cactgcgctg 960

atggatggtc gtctggacgc tgttgtattc actggtggta tcggtgaaaa tgccgcaatg 1020

gttcgtgaac tgtctctggg caaactgggc gtgctgggct ttgaagttga tcatgaacgc 1080

aacctggctg cacgtttcgg caaatctggt ttcatcaaca aagaaggtac ccgtcctgcg 1140

gtggttatcc caaccaacga agaactggtt atcgcgcaag acgcgagccg cctgactgcc 1200

tga 1203

<210> 36
<211> 2145
<212> DNA
<213> Escherichia coli

<400> 36
gtgtcccgta ttattatgct gatccctacc ggaaccagcg tcggtctgac cagcgtcagc 60

cttggcgtga tccgtgcaat ggaacgcaaa ggcgttcgtc tgagcgtttt caaacctatc 120

gctcagccgc gtaccggtgg cgatgcgccc gatcagacta cgactatcgt gcgtgcgaac 180

tcttccacca cgacggccgc tgaaccgctg aaaatgagct acgttgaagg tctgctttcc 240

agcaatcaga aagatgtgct gatggaagag atcgtcgcaa actaccacgc taacaccaaa 300

gacgctgaag tcgttctggt tgaaggtctg gtcccgacac gtaagcacca gtttgcccag 360

tctctgaact acgaaatcgc taaaacgctg aatgcggaaa tcgtcttcgt tatgtctcag 420

ggcactgaca ccccggaaca gctgaaagag cgtatcgaac tgacccgcaa cagcttcggc 480

ggtgccaaaa acaccaacat caccggcgtt atcgttaaca aactgaacgc accggttgat 540

gaacagggtc gtactcgccc ggatctgtcc gagattttcg acgactcttc caaagctaaa 600

gtaaacaatg ttgatccggc gaagctgcaa gaatccagcc cgctgccggt tctcggcgct 660

gtgccgtgga gctttgacct gatcgcgact cgtgcgatcg atatggctcg ccacctgaat 720

gcgaccatca tcaacgaagg cgacatcaat actcgccgcg ttaaatccgt cactttctgc 780

gcacgcagca ttccgcacat gctggagcac ttccgtgccg gttctctgct ggtgacttcc 840

gcagaccgtc ctgacgtgct ggtggccgct tgcctggcag ccatgaacgg cgtagaaatc 900

ggtgccctgc tgctgactgg cggttacgaa atggacgcgc gcatttctaa actgtgcgaa 960

cgtgctttcg ctaccggcct gccggtattt atggtgaaca ccaacacctg gcagacctct 1020

ctgagcctgc agagcttcaa cctggaagtt ccggttgacg atcacgaacg tatcgagaaa 1080

gttcaggaat acgttgctaa ctacatcaac gctgactgga tcgaatctct gactgccact 1140

tctgagcgca gccgtcgtct gtctccgcct gcgttccgtt atcagctgac tgaacttgcg 1200

cgcaaagcgg gcaaacgtat cgtactgccg gaaggtgacg aaccgcgtac cgttaaagca 1260

gccgctatct gtgctgaacg tggtatcgca acttgcgtac tgctgggtaa tccggcagag 1320

atcaaccgtg ttgcagcgtc tcagggtgta gaactgggtg cagggattga aatcgttgat 1380

ccagaagtgg ttcgcgaaag ctatgttggt cgtctggtcg aactgcgtaa gaacaaaggc 1440

atgaccgaaa ccgttgcccg cgaacagctg gaagacaacg tggtgctcgg tacgctgatg 1500

ctggaacagg atgaagttga tggtctggtt tccggtgctg ttcacactac cgcaaacacc 1560

atccgtccgc cgctgcagct gatcaaaact gcaccgggca gctccctggt atcttccgtg 1620

ttcttcatgc tgctgccgga acaggtttac gtttacggtg actgtgcgat caacccggat 1680

ccgaccgctg aacagctggc agaaatcgcg attcagtccg ctgattccgc tgcggccttc 1740

ggtatcgaac cgcgcgttgc tatgctctcc tactccaccg gtacttctgg tgcaggtagc 1800

gacgtagaaa aagttcgcga agcaactcgt ctggcgcagg aaaaacgtcc tgacctgatg 1860

atcgacggtc cgctgcagta cgacgctgcg gtaatggctg acgttgcgaa atccaaagcg 1920

ccgaactctc cggttgcagg tcgcgctacc gtgttcatct tcccggatct gaacaccggt 1980

aacaccacct acaaagcggt acagcgttct gccgacctga tctccatcgg gccgatgctg 2040

cagggtatgc gcaagccggt taacgacctg tcccgtggcg cactggttga cgatatcgtc 2100

tacaccatcg cgctgactgc gattcagtct gcacagcagc agtaa 2145

<210> 37
<211> 2676
<212> DNA
<213> Escherichia coli

<400> 37
atggctgtta ctaatgtcgc tgaacttaac gcactcgtag agcgtgtaaa aaaagcccag 60

cgtgaatatg ccagtttcac tcaagagcaa gtagacaaaa tcttccgcgc cgccgctctg 120

gctgctgcag atgctcgaat cccactcgcg aaaatggccg ttgccgaatc cggcatgggt 180

atcgtcgaag ataaagtgat caaaaaccac tttgcttctg aatatatcta caacgcctat 240
aaagatgaaa aaacctgtgg tgttctgtct gaagacgaca cttttggtac catcactatc 300
gctgaaccaa tcggtattat ttgcggtatc gttccgacca ctaacccgac ttcaactgct 360
atcttcaaat cgctgatcag tctgaagacc cgtaacgcca ttatcttctc cccgcacccg 420
cgtgcaaaag atgccaccaa caaagcggct gatatcgttc tgcaggctgc tatcgctgcc 480
ggtgctccga aagatctgat cggctggatc gatcaacctt ctgttgaact gtctaacgca 540
ctgatgcacc acccagacat caacctgatc ctcgcgactg gtggtccggg catggttaaa 600
gccgcataca gctccggtaa accagctatc ggtgtaggcg cgggcaacac tccagttgtt 660
atcgatgaaa ctgctgatat caaacgtgca gttgcatctg tactgatgtc caaaaccttc 720
gacaacggcg taatctgtgc ttctgaacag tctgttgttg ttgttgactc tgtttatgac 780
gctgtacgtg aacgttttgc aacccacggc ggctatctgt tgcagggtaa agagctgaaa 840
gctgttcagg atgttatcct gaaaaacggt gcgctgaacg cggctatcgt tggtcagcca 900
gcctataaaa ttgctgaact ggcaggcttc tctgtaccag aaaacaccaa gattctgatc 960
ggtgaagtga ccgttgttga tgaaagcgaa ccgttcgcac atgaaaaact gtccccgact 1020
ctggcaatgt accgcgctaa agatttcgaa gacgcggtag aaaaagcaga gaaactggtt 1080
gctatgggcg gtatcggtca tacctcttgc ctgtacactg accaggataa ccaaccggct 1140
cgcgtttctt acttcggtca gaaaatgaaa acggcgcgta tcctgattaa caccccagcg 1200
tctcagggtg gtatcggtga cctgtataac ttcaaactcg caccttccct gactctgggt 1260
tgtggttctt ggggtggtaa ctccatctct gaaaacgttg gtccgaaaca cctgatcaac 1320
aagaaaaccg ttgctaagcg agctgaaaac atgttgtggc acaaacttcc gaaatctatc 1380
tacttccgcc gtggctccct gccaatcgcg ctggatgaag tgattactga tggccacaaa 1440
cgtgcgctca tcgtgactga ccgcttcctg ttcaacaatg gttatgctga tcagatcact 1500
tccgtactga aagcagcagg cgttgaaact gaagtcttct tcgaagtaga agcggacccg 1560
accctgagca tcgttcgtaa aggtgcagaa ctggcaaact ccttcaaacc agacgtgatt 1620
atcgcgctgg gtggtggttc cccgatggac gccgcgaaga tcatgtgggt tatgtacgaa 1680
catccggaaa ctcacttcga agagctggcg ctgcgcttta tggatatccg taaacgtatc 1740
tacaagttcc cgaaaatggg cgtgaaagcg aaaatgatcg ctgtcaccac cacttctggt 1800
acaggttctg aagtcactcc gtttgcggtt gtaactgacg acgctactgg tcagaaatat 1860
ccgctggcag actatgcgct gactccggat atggcgattg tcgacgccaa cctggttatg 1920
gacatgccga agtccctgtg tgctttcggt ggtctggacg cagtaactca cgccatggaa 1980
gcttatgttt ctgtactggc atctgagttc tctgatggtc aggctctgca ggcactgaaa 2040

ctgctgaaag aatatctgcc agcgtcctac cacgaagggt ctaaaaatcc ggtagcgcgt 2100

gaacgtgttc acagtgcagc gactatcgcg ggtatcgcgt ttgcgaacgc cttcctgggt 2160

gtatgtcact caatggcgca caaactgggt tcccagttcc atattccgca cggtctggca 2220

aacgccctgc tgatttgtaa cgttattcgc tacaatgcga acgacaaccc gaccaagcag 2280

actgcattca gccagtatga ccgtccgcag gctcgccgtc gttatgctga aattgccgac 2340

cacttgggtc tgagcgcacc gggcgaccgt actgctgcta agatcgagaa actgctggca 2400

tggctggaaa cgctgaaagc tgaactgggt attccgaaat ctatccgtga agctggcgtt 2460

caggaagcag acttcctggc gaacgtggat aaactgtctg aagatgcatt cgatgaccag 2520

tgcaccggcg ctaacccgcg ttacccgctg atctccgagc tgaaacagat tctgctggat 2580

acctactacg gtcgtgatta tgtagaaggt gaaactgcag cgaagaaaga agctgctccg 2640

gctaaagctg agaaaaaagc gaaaaaatcc gcttaa 2676

<210> 38
<211> 1809
<212> DNA
<213> Escherichia coli

<400> 38
gtgcaaacct ttcaagccga tcttgccatt gtaggcgccg gtggcgcggg attacgtgct 60

gcaattgctg ccgcgcaggc aaatccgaat gcaaaaatcg cactaatctc aaaagtatac 120

ccgatgcgta gccataccgt tgctgcagaa gggggctccg ccgctgtcgc gcaggatcat 180

gacagcttcg aatatcactt tcacgataca gtagcgggtg gcgactggtt gtgtgagcag 240

gatgtcgtgg attatttcgt ccaccactgc ccaaccgaaa tgacccaact ggaactgtgg 300

ggatgcccat ggagccgtcg cccggatggt agcgtcaacg tacgtcgctt cggcggcatg 360

aaaatcgagc gcacctggtt cgccgccgat aagaccggct tccatatgct gcacacgctg 420

ttccagacct ctctgcaatt cccgcagatc cagcgttttg acgaacattt cgtgctggat 480

attctggttg atgatggtca tgttcgcggc ctggtagcaa tgaacatgat ggaaggcacg 540

ctggtgcaga tccgtgctaa cgcggtcgtt atggctactg gcggtgcggg tcgcgtttat 600

cgttacaaca ccaacggcgg catcgttacc ggtgacggta tgggtatggc gctaagccac 660

ggcgttccgc tgcgtgacat ggaattcgtt cagtatcacc caaccggtct gccaggttcc 720

ggtatcctga tgaccgaagg ttgccgcggt gaaggcggta ttctggtcaa caaaaatggc 780

taccgttatc tgcaagatta cggcatgggc ccggaaactc cgctgggcga gccgaaaaac 840

aaatatatgg aactgggtcc acgcgacaaa gtctctcagg ccttctggca cgaatggcgt 900

aaaggcaaca ccatctccac gccgcgtggc gatgtggttt atctcgactt gcgtcacctc 960

ggcgagaaaa aactgcatga acgtctgccg ttcatctgcg aactggcgaa agcgtacgtt 1020

ggcgtcgatc cggttaaaga accgattccg gtacgtccga ccgcacacta caccatgggc 1080

ggtatcgaaa ccgatcagaa ctgtgaaacc cgcattaaag gtctgttcgc cgtgggtgaa 1140

tgttcctctg ttggtctgca cggtgcaaac cgtctgggtt ctaactccct ggcggaactg 1200

gtggtcttcg gccgtctggc cggtgaacaa gcgacagagc gtgcagcaac tgccggtaat 1260

ggcaacgaag cggcaattga agcgcaggca gctggcgttg aacaacgtct gaaagatctg 1320

gttaaccagg atggcggcga aaactgggcg aagatccgcg acgaaatggg cctggctatg 1380

gaagaaggct gcggtatcta ccgtacgccg gaactgatgc agaaaaccat cgacaagctg 1440

gcagagctgc aggaacgctt caagcgcgtg cgcatcaccg acacttccag cgtgttcaac 1500

accgacctgc tctacaccat tgaactgggc cacggtctga acgttgctga atgtatggcg 1560

cactccgcaa tggcacgtaa agagtcccgc ggcgcgcacc agcgtctgga cgaaggttgc 1620

accgagcgtg acgacgtcaa cttcctcaaa cacaccctcg ccttccgcga tgctgatggc 1680

acgactcgcc tggagtacag cgacgtgaag attactacgc tgccgccagc taaacgcgtt 1740

tacggtggcg aagcggatgc agccgataag gcggaagcag ccaataagaa ggagaaggcg 1800

aatggctga 1809

<210> 39
<211> 735
<212> DNA
<213> Escherichia coli

<400> 39
atggctgaga tgaaaaacct gaaaattgag gtggtgcgct ataacccgga agtcgatacc 60

gcaccgcata gcgcattcta tgaagtgcct tatgacgcaa ctacctcatt actggatgcg 120

ctgggctaca tcaaagacaa cctggcaccg gacctgagct accgctggtc ctgccgtatg 180

gcgatttgtg gttcctgcgg catgatggtt aacaacgtgc caaaactggc atgtaaaacc 240

ttcctgcgtg attacaccga cggtatgaag gttgaagcgt tagctaactt cccgattgaa 300

cgcgatctgg tggtcgatat gacccacttc atcgaaagtc tggaagcgat caaaccgtac 360

atcatcggca actcccgcac cgcggatcag ggtactaaca tccagacccc ggcgcagatg 420

gcgaagtatc accagttctc cggttgcatc aactgtggtt tgtgctacgc cgcgtgcccg 480

cagtttggcc tgaacccaga gttcatcggt ccggctgcca ttacgctggc gcatcgttat 540

aacgaagata gccgcgacca cggtaagaag gagcgtatgg cgcagttgaa cagccagaac 600

ggcgtatgga gctgtacttt cgtgggctac tgctccgaag tctgcccgaa acacgtcgat 660

ccggctgcgg ccattcagca gggcaaagta gaaagttcga aagactttct tatcgcgacc 720

ctgaaaccac gctaa 735

<210> 40

<211> 396
<212> DNA
<213> Escherichia coli

<400> 40
atgacgacta aacgtaaacc gtatgtacgg ccaatgacgt ccacctggtg gaaaaaattg 60

ccgttttatc gcttttacat gctgcgcgaa ggcacggcgg ttccggctgt gtggttcagc 120

attgaactga ttttcgggct gtttgccctg aaaaatggcc cggaagcctg ggcgggattc 180

gtcgactttt tacaaaaccc ggttatcgtg atcattaacc tgatcactct ggcggcagct 240

ctgctgcaca ccaaaacctg gtttgaactg gcaccgaaag cggccaatat cattgtaaaa 300

gacgaaaaaa tgggaccaga gccaattatc aaaagtctct gggcggtaac tgtggttgcc 360

accatcgtaa tcctgtttgt tgccctgtac tggtaa 396

<210> 41
<211> 360
<212> DNA
<213> Escherichia coli

<400> 41
atgattaatc caaatccaaa gcgttctgac gaaccggtat tctggggcct cttcggggcc 60

ggtggtatgt ggagcgccat cattgcgccg gtgatgatcc tgctggtggg tattctgctg 120

ccactggggt tgtttccggg tgatgcgctg agctacgagc gcgttctggc gttcgcgcag 180

agcttcattg gtcgcgtatt cctgttcctg atgatcgttc tgccgctgtg gtgtggttta 240

caccgtatgc accacgcgat gcacgatctg aaaatccacg tacctgcggg caaatgggtt 300

ttctacggtc tggctgctat cctgacagtt gtcacgctga ttggtgtcgt tacaatctaa 360

<210> 42
<211> 990
<212> DNA
<213> Escherichia coli

<400> 42
atgaaactcg ccgtttatag cacaaaacag tacgacaaga agtacctgca acaggtgaac 60

gagtcctttg gctttgagct ggaatttttt gactttctgc tgacggaaaa aaccgctaaa 120

actgccaatg gctgcgaagc ggtatgtatt ttcgtaaacg atgacggcag ccgcccggtg 180

ctggaagagc tgaaaaagca cggcgttaaa tatatcgccc tgcgctgtgc cggtttcaat 240

aacgtcgacc ttgacgcggc aaaagaactg gggctgaaag tagtccgtgt tccagcctat 300

gatccagagg ccgttgctga acacgccatc ggtatgatga tgacgctgaa ccgccgtatt 360

caccgcgcgt atcagcgtac ccgtgatgct aacttctctc tggaaggtct gaccggcttt 420

actatgtatg gcaaaacggc aggcgttatc ggtaccggta aaatcggtgt ggcgatgctg 480

cgcattctga aaggttttgg tatgcgtctg ctggcgttcg atccgtatcc aagtgcagcg 540

gcgctggaac tcggtgtgga gtatgtcgat ctgccaaccc tgttctctga atcagacgtt 600

atctctctgc actgcccgct gacaccggaa aactatcatc tgttgaacga agccgccttc 660

gaacagatga aaaatggcgt gatgatcgtc aataccagtc gcggtgcatt gattgattct 720

caggcagcaa ttgaagcgct gaaaaatcag aaaattggtt cgttgggtat ggacgtgtat 780

gagaacgaac gcgatctatt ctttgaagat aaatccaacg acgtgatcca ggatgacgta 840

ttccgtcgcc tgtctgcctg ccacaacgtg ctgtttaccg ggcaccaggc attcctgaca 900

gcagaagctc tgaccagtat ttctcagact acgctgcaaa acttaagcaa tctggaaaaa 960

ggcgaaacct gcccgaacga actggtttaa 990

<210> 43
<211> 459
<212> DNA
<213> Escherichia coli

<400> 43
atggaactga cgactcgcac tttacctgcg cggaaacata ttgcgctggt ggcacacgat 60

cactgcaaac aaatgctgat gagctgggtg gaacggcatc aaccgttact ggaacaacac 120

gtactgtatg caacaggcac taccggtaac ttaatttccc gcgcgaccgg catgaacgtc 180

aacgcgatgt tgagtggccc aatgggggt gaccagcagg ttggcgcatt gatctcagaa 240

gggaaaattg atgtattgat tttcttctgg gatccactaa atgccgtgcc gcacgatcct 300

gacgtgaaag ccttgctgcg tctggcgacg gtatggaaca ttccggtcgc caccaacgtg 360

gcaacggcag acttcataat ccagtcgccg catttcaacg acgcggtcga tattctgatc 420

cccgattatc agcgttatct cgcggaccgt ctgaagtaa 459

<210> 44
<211> 741
<212> DNA
<213> Escherichia coli

<400> 44
atgtcagtta ttggtcgcat tcactccttt gaatcctgtg gaaccgtaga cggcccaggt 60

attcgcttta tcaccttttt ccagggctgc ctgatgcgct gcctgtattg tcataaccgc 120

gacacctggg acacgcatgg cggtaaagaa gttaccgttg aagatttgat gaaggaagtg 180

gtgacctatc gccactttat gaacgcttcc ggcggcggcg ttaccgcatc cggcggtgaa 240

gcaatcctgc aagctgagtt tgttcgtgac tggttccgcg cctgcaaaaa agaaggcatt 300

catacctgtc tggacaccaa cggttttgtt cgtcgttacg atccggtgat tgatgaactg 360

ctggaagtaa ccgacctggt aatgctcgat ctcaaacaga tgaacgacga gatccaccaa 420

aatctggttg gagtttccaa ccaccgcacg ctggagttcg ctaaatatct ggcgaacaaa 480

aatgtgaagg tgtggatccg ctacgttgtt gtcccaggct ggtctgacga tgacgattca 540

gcgcatcgcc tcggtgaatt tacccgtgat atgggcaacg ttgagaaaat cgagcttctc 600

ccctaccacg agctgggcaa acacaaatgg gtggcaatgg gtgaagagta caaactcgac 660

ggtgttaaac caccgaagaa agagaccatg gaacgcgtga aaggcattct tgagcagtac 720

ggtcataagg taatgttcta a 741

<210> 45
<211> 2283
<212> DNA
<213> Escherichia coli

<400> 45
atgtccgagc ttaatgaaaa gttagccaca gcctgggaag gttttaccaa aggtgactgg 60

cagaatgaag taaacgtccg tgacttcatt cagaaaaact acactccgta cgagggtgac 120

gagtccttcc tggctggcgc tactgaagcg accaccaccc tgtgggacaa agtaatggaa 180

ggcgttaaac tggaaaaccg cactcacgcg ccagttgact ttgacaccgc tgttgcttcc 240

accatcacct ctcacgacgc tggctacatc aacaagcagc ttgagaaaat cgttggtctg 300

cagactgaag ctccgctgaa acgtgctctt atcccgttcg gtggtatcaa aatgatcgaa 360

ggttcctgca aagcgtacaa ccgcgaactg gatccgatga tcaaaaaaat cttcactgaa 420

taccgtaaaa ctcacaacca gggcgtgttc gacgtttaca ctccggacat cctgcgttgc 480

cgtaaatctg gtgttctgac cggtctgcca gatgcatatg gccgtggccg tatcatcggt 540

gactaccgtc gcgttgcgct gtacggtatc gactacctga tgaaagacaa actggcacag 600

ttcacttctc tgcaggctga tctggaaaac ggcgtaaacc tggaacagac tatccgtctg 660

cgcgaagaaa tcgctgaaca gcaccgcgct ctgggtcaga tgaaagaaat ggctgcgaaa 720

tacggctacg acatctctgg tccggctacc aacgctcagg aagctatcca gtggacttac 780

ttcggctacc tggctgctgt taagtctcag aacggtgctg caatgtcctt cggtcgtacc 840

tccaccttcc tggatgtgta catcgaacgt gacctgaaag ctggcaagat caccgaacaa 900

gaagcgcagg aaatggttga ccacctggtc atgaaactgc gtatggttcg cttcctgcgt 960

actccggaat acgatgaact gttctctggc gacccgatct gggcaaccga atctatcggt 1020

ggtatgggcc tcgacggtcg taccctggtt accaaaaaca gcttccgttt cctgaacacc 1080

ctgtacacca tgggtccgtc tccggaaccg aacatgacca ttctgtggtc tgaaaaactg 1140

ccgctgaact tcaagaaatt cgccgctaaa gtgtccatcg acacctcttc tctgcagtat 1200

gagaacgatg acctgatgcg tccggacttc aacaacgatg actacgctat tgcttgctgc 1260

gtaagcccga tgatcgttgg taaacaaatg cagttcttcg gtgcgcgtgc aaacctggcg 1320

aaaaccatgc tgtacgcaat caacggcggc gttgacgaaa aactgaaaat gcaggttggt 1380

ccgaagtctg aaccgatcaa aggcgatgtc ctgaactatg atgaagtgat ggagcgcatg 1440

gatcacttca tggactggct ggctaaacag tacatcactg cactgaacat catccactac 1500

atgcacgaca agtacagcta cgaagcctct ctgatggcgc tgcacgaccg tgacgttatc 1560

cgcaccatgg cgtgtggtat cgctggtctg tccgttgctg ctgactccct gtctgcaatc 1620

aaatatgcga aagttaaacc gattcgtgac gaagacggtc tggctatcga cttcgaaatc 1680

gaaggcgaat acccgcagtt tggtaacaat gatccgcgtg tagatgacct ggctgttgac 1740

ctggtagaac gtttcatgaa gaaaattcag aaactgcaca cctaccgtga cgctatcccg 1800

actcagtctg ttctgaccat cacttctaac gttgtgtatg gtaagaaaac gggtaacacc 1860

ccagacggtc gtcgtgctgg cgcgccgttc ggaccgggtg ctaacccgat gcacggtcgt 1920

gaccagaaag gtgcagtagc ctctctgact tccgttgcta aactgccgtt tgcttacgct 1980

aaagatggta tctcctacac cttctctatc gttccgaacg cactgggtaa agacgacgaa 2040

gttcgtaaga ccaacctggc tggtctgatg gatggttact tccaccacga agcatccatc 2100

gaaggtggtc agcacctgaa cgttaacgtg atgaaccgtg aaatgctgct cgacgcgatg 2160

gaaaacccgg aaaaatatcc gcagctgacc atccgtgtat ctggctacgc agtacgtttc 2220

aactcgctga ctaaagaaca gcagcaggac gttattactc gtaccttcac tcaatctatg 2280

taa 2283

<210> 46
<211> 1413
<212> DNA
<213> Escherichia coli

<400> 46
atggtagatc aggtaaaagt cgttgccgat gatcaggctc cggctgaaca gtcgctacgg 60

cgcaatctca caaaccgaca tattcagctt attgccattg gcggtgccat tggtacgggg 120

ttgtttatgg ggtctggcaa aacgattagc cttgccgggc cgtcgatcat tttcgtttat 180

atgatcattg gttttatgct ctttttcgtg atgcgggcaa tgggggaatt gctgctttcg 240

aatctggaat acaaatcttt tagtgacttc gcttccgatt tactcgggcc gtgggcagga 300

tatttcaccg gctggactta ctggttctgc tgggttgtaa ccggtatggc agacgtggtg 360

gcgatcacgg cttatgctca gttctggttc cccgatctct ccgactgggt cgcctcgctg 420

gcggtgatag tgctgctgct gacgctcaat ctcgccaccg tgaaaatgtt cggtgagatg 480

gagttctggt ttgcgatgat caaaatcgtc gccattgtgt cgctgattgt cgtcggcctg 540

gtcatggtgg cgatgcactt tcagtcaccg actggtgtgg aagcgtcatt cgcgcatttg 600

tggaatgacg gcggctggtt cccgaaaggt ttaagtggct tctttgccgg attccagata 660

gcggttttcg ctttcgtggg gattgagctg gtaggtacaa cagctgcgga aaccaaagat 720

ccagagaaat cactgccacg cgcgattaac tccattccga tccgtatcat tatgttctac 780

gtcttcgcgc tgattgtgat tatgtccgtg acgccgtgga gttcggtagt cccggagaaa 840

agcccgtttg ttgaactgtt cgtgttggta gggctgcctg ctgccgcaag cgtgatcaac 900

tttgtggtgc tgacctctgc ggcgtcttcc gctaacagcg gcgtcttctc taccagccgt 960

atgctgtttg gtctggcgca ggaaggtgtg gcaccgaaag cgttcgctaa actttctaag 1020

cgcgcagtac ccgcgaaagg gctgacgttc tcgtgtatct gtctgctcgg cggcgtggtg 1080

atgttgtatg tgaatcctag tgtgattggc gcgttcacga tgattacaac cgtttccgcg 1140

attctgttta tgttcgtctg gacgattatc ctttgctcgt accttgtgta tcgcaaacag 1200

cgtcctcatc tacatgagaa gtcgatctac aagatgccgc tcggcaagct gatgtgctgg 1260

gtatgtatgg cgttctttgt gttcgtggtc gtgttgctga cactggaaga tgacactcgc 1320

caggcgctgc tggttacccc gctgtggttt atcgcgctgg ggttgggctg gctgtttatt 1380

ggtaagaagc gggctgctga actgcggaaa taa 1413

<210> 47
<211> 1071
<212> DNA
<213> Escherichia coli

<400> 47
atgacccgtc cgatacaggc cagcctcgat ctgcaggcat taaaacagaa tctgtccatt 60

gtccgccagg ccgcgacgca cgcgcgcgtc tggtcggtgg taaaagcgaa cgcttacggg 120

catggtattg agcgtatctg gagcgcgatc ggggccaccg atggctttgc attgcttaac 180

ctggaagagg caataacgtt acgtgagcgc ggctggaaag gaccgatcct gatgctggaa 240

ggatttttcc atgctcagga tctggagatt tatgaccagc accgcctgac cacctgcgta 300

cacagcaact ggcagctcaa agcactgcaa aatgcgcggc taaaagcacc gttggatatt 360

tatcttaaag tgaacagtgg gatgaatcgg ttgggcttcc agcccgatcg cgtgcttacc 420

gtctggcagc agttgcgggc aatggcgaat gttggcgaaa tgaccctgat gtcgcatttt 480

gccgaagcgg aacatcctga tggaatttcc ggccggatgg cgcgtattga gcaggcggcg 540

gaggggctgg agtgtcggcg ttcgttgtcc aattcggcgg cgactctgtg gcacccggaa 600

gcgcattttg actgggttcg gcctggcatt attttgtatg gccgttcgcc gtccggtcag 660

tggcgtgata tcgccaatac cggattacgt ccggtgatga cgctaagcag tgagattatt 720

ggtgtccaga cgctaaaagc gggcgagcgt gtgggctacg gcggtcgcta tactgcgcgc 780

gatgaacagc gaatcggcat tgtcgccgca gggtacgccg acggttatcc gcgccacgcc 840

ttaaccggta cccctgtttt agtggacggc gtgcgcacca tgacggtggg gaccgtctcg 900

atggatatgc tagcggtcga tttaacgcct tgcccgcagg cgggtattgg tacgccggtt 960

gagctgtggg gcaaggagat caaaattgat gatgtcgccg ccgctgccgg aacggtgggc 1020

```
tatgagttga tgtgcgcgct ggccgtacgc gtcccggttg tgacggtgta a                1071
```

## Description

### Field of invention

The present invention relates to a microorganism genetically modified for the improved production of alanine and to a method for the improved production of alanine using said microorganism.

### Background of the invention

L-alanine is a non-essential amino acid with numerous industrial applications, in particular in the food industry as a flavor enhancer and/or nutritional supplement as well as in the pharmaceutical industry (e.g. in the context of nutrition therapy and in the synthesis of vitamin B6). It is an odorless, sweet, white solid crystal that is soluble in water and ethanol but not in ether. The global alanine market in 2020 is valued at more than 290 million USD, with production estimated at 500 tons per year (The Expresswire, 2020; Wendisch, 2014).

L-alanine may be manufactured via chemical synthesis (e.g. via Strecker synthesis or the Bucherer-Bergs method variant), enzymatic conversion, or fermentation. That said, chemical methods are of little industrial interest as both D- and L- enantiomers are generated in equimolar amounts, while enzymatic and fermentation methods produce only one enantiomer. L-alanine is generally produced on an industrial scale via the enzymatic decarboxylation of L-aspartic acid using L-aspartate $\beta$-decarboxylase from *Pseudomonas dacunhae* as such or using P. *dacunhae* suspensions or cells immobilized by $\kappa$-carragenan as a biocatalyst (Leuchtenberger et al., 2005). The initial substrate, L-aspartic acid, is notably also obtained by enzymatic conversion of ammonium fumarate (Sato et al., 1982; Leuchtenberger et al., 2005). While yields of up to 90% can be obtained, the production of fumarate is dependent on petroleum and remains costly. The cost of the L-aspartic acid intermediate is also high. More recently, enzymatic conversion of L-alanine from D-glucose and ammonium sulfate has been described (Gmelch et al., 2019). However, this method relies upon six different enzymes and production yield remains largely inferior to that obtained with immobilized P. *dacunhae* cells.

Fermentation by microorganisms represents an interesting alternative to the more traditional chemical and enzymatic means of generating alanine, and furthermore provides a useful way of using abundant, renewable, and/or inexpensive materials as the main source of carbon. While most microorganisms naturally produce alanine for biosynthesis (e.g. using a glutamate-pyruvate transaminase or from pyruvate and ammonia using an NADH-linked alanine dehydrogenase), fermentations are slow and yields remain low, in particular due to the production of co-products that are undesired in an industrial setting (Zhang et al., 2007). In view of improving alanine production, several microorganisms genetically modified for the production of alanine have been described to date. Such microorganisms generally overexpress AlaE (also referred to as YgaW), which is the major L-alanine exporter, and one or more heterologous genes involved in L-alanine synthesis. As an example, CN108060114 discloses an *E. coli* microorganism producing fumaric acid which it can then use to produce L-alanine via the enzymatic decarboxylation of L-aspartic acid described above and further comprises an overexpression of the *alaE* gene. WO2012172822 discloses microorganisms overexpressing both the *Bacillus sphaericus* gene *alaD* coding for an alanine dehydrogenase and the *E. coli alaE* gene.

Nevertheless, there remains a need for improved microorganisms that are able to produce alanine with high levels of production, titer and yield, in particular from an inexpensive and/or abundant carbon source such as glucose. There also remains a need for novel methods for the production of alanine at a reduced cost, ideally wherein the production, titer, and/or yield of alanine is at least similar to that obtained with current methods.

### Brief description of the invention

The present invention concerns a microorganism genetically modified for the production of alanine and methods for the production of alanine using said microorganism. The microorganism genetically modified for the production of alanine notably expresses a heterologous *alaD* gene coding an alanine dehydrogenase and has reduced Lrp transcription factor activity and/or expression.

Indeed, the inventors have surprisingly found that such a microorganism shows improved production of alanine, despite the decreased expression of the *alaE* gene that is associated with reduced Lrp transcription factor activity and/or expression. Indeed, Lrp is known to positively regulate expression of the *alaE* gene (Ihara et al., 2017).

Preferably, the microorganism comprises an *lrp* gene coding for an Lrp* mutant having reduced transcription factor activity.

Preferably, the Lrp* mutant comprises at least one mutation selected from the group consisting of L108F, L74F, F113C, and 123PD, wherein the positions of the amino acid residues correspond to those provided in SEQ ID NO: 1.

Preferably, the microorganism comprises at least a partial deletion of the *lrp* gene, preferably a complete deletion of the *lrp* gene.

Preferably, the microorganism further comprises an overexpression of the *yddG* gene.

Preferably, the microorganism further comprises expression of an *alaE* gene coding an L-alanine exporter at a level similar to that of the corresponding microorganism which does not comprise the genetic modifications as provided herein, preferably by modifying the *alaE* promoter or by increasing the number of copies of the *alaE* gene present in the microorganism.

Preferably, the microorganism expresses the *alaD* gene of *Geobacillus stearothermophilus, Klebsiella aerogenes* or *Archaeoglobus fulgidus.*

Preferably, the *alaD* gene codes the alanine dehydrogenase of SEQ ID NO: 15, 17, 19, 23, or 27.

Preferably, the microorganism further comprises a deletion of at least one gene selected from the group consisting of *ackA-pta, ldhA, adhE, frdABCD, mgsA,* and *pflAB.*

Preferably, the microorganism comprises the deletion of genes *ackA-pta, ldhA, adhE, frdABCD, mgsA,* and *pflAB.*

Preferably, the microorganism further comprises a deletion of the *cycA* and/or *dadX* gene(s).

Preferably, the microorganism belongs to the family of bacteria Enterobacteriaceae, Clostridiaceae, Bacillaceae, Streptomycetaceae, or Corynebacteriaceae, or to the family of yeasts Saccharomycetaceae.

Preferably, said Enterobacteriaceae bacterium is *Escherichia coli* or *Klebsiella pneumoniae,* said Clostridiaceae bacterium is *Clostridium acetobutylicum,* said Corynebacteriaceae bacterium is *Corynebacterium glutamicum,* or said Saccharomycetaceae yeast is *Saccharomyces cerevisiae,* more preferably said microorganism is *Escherichia coli.*

The present invention further comprises a method for the production of alanine comprising the steps of:

a) culturing a microorganism genetically modified for the production of alanine described in any of the embodiments provided herein in an appropriate culture medium comprising a source of carbon, and
b) recovering alanine from the culture medium.

Preferably, the source of carbon is selected from arabinose, fructose, galactose, glucose, lactose, maltose, sucrose, xylose, and any combination thereof.

**Detailed description**

Before describing the present invention in detail, it is to be understood that the invention is not limited to particularly exemplified microorganisms and/or methods and may, of course, vary. It is also to be understood that the terminology used herein is for the purpose of describing particular embodiments of the invention only, and is not intended to be limiting. The invention will be limited only by the appended claims.

All publications, patents and patent applications cited herein, whether supra or infra, are hereby incorporated by reference in their entirety. Furthermore, the practice of the present invention employs, unless otherwise indicated, conventional microbiological and molecular biological techniques that are within the skill of the art. Such techniques are well-known to the skilled person, and are fully explained in the literature. See, for example, Prescott *et al.* (1999) and Sambrook and Russell (2001).

Unless defined otherwise, all technical and scientific terms used herein have the same meanings as commonly understood by one of ordinary skill in the art to which this invention belongs. Although any materials and methods similar or equivalent to those described herein can be used to practice or test the present invention, preferred materials and methods are provided.

It must be noted that as used herein and in the appended claims, the singular forms "a," "an," and "the," include the plural reference unless the context clearly dictates otherwise. Thus, for example, a reference to "a microorganism" includes a plurality of such microorganisms, and a reference to "an endogenous gene" is a reference to one or more endogenous genes, and so forth.

The terms "comprise," "comprises," and "comprising" are used in an inclusive sense, i.e. to specify the presence of the stated features but not to preclude the presence or addition of further features in various embodiments of the invention.

A first aspect of the invention relates to a microorganism genetically modified for the production of alanine.

The term "microorganism," as used herein, refers to a living microscopic organism, which may be a single cell or a multicellular organism and which can generally be found in nature. In the present context, the microorganism is preferably a bacterium, yeast or fungus. Preferably, the microorganism of the invention is selected from the Enterobacteriaceae, Clostridiaceae, Bacillaceae, Streptomycetaceae, or Corynebacteriaceae family or from among yeast, more preferably from the Saccharomycetaceae family. More preferably, the microorganism of the invention is a species of *Escherichia, Klebsiella, Thermoanaerobacterium, Clostridium, Corynebacterium* or *Saccharomyces.* Even more preferably, said Enterobacteriaceae bacterium is *Escherichia coli* or *Klebsiella pneumoniae,* said Clostridiaceae bacterium is *Clostridium acetobutylicum,* said Corynebacteriaceae bacterium is *Corynebacterium glutamicum,* or said Saccharomycetaceae yeast is *Saccharomyces cerevisiae.* Most preferably, the microorganism of the invention is *Escherichia coli.*

The terms "recombinant microorganism" or "microorganism genetically modified" are used interchangeably herein and refer to a microorganism or a strain of microorganism that has been genetically modified or genetically engineered. This means, according to the usual meaning of these terms, that the microorganism of the invention is not found in nature and is genetically modified when compared to the "parental" microorganism from which it is derived. The "parental" microorganism may occur in nature (i.e. a wild-type microorganism) or may have been previously modified. The recombinant microorganism of the invention may notably be modified by the introduction, deletion and/or modification of genetic elements. Such modifications can be performed, for example, by genetic engineering, by adaptation, wherein a microorganism is cultured in conditions that apply a specific stress on the microorganism and induce mutagenesis, and/or by forcing the development and evolution of metabolic pathways by combining directed mutagenesis and evolution under specific selection pressure.

A microorganism may notably be modified to modulate the expression level of an endogenous gene or the activity of the corresponding enzyme or transcription factor. The term "endogenous gene" means that the gene was present in the microorganism before any genetic modification. Endogenous genes may be overexpressed by introducing heterologous sequences in addition to, or to replace, endogenous regulatory elements. Endogenous gene expression levels, protein expression levels, or the activity of the encoded protein, can also be increased or attenuated by introducing mutations into the coding sequence of a gene or into non-coding sequences. These mutations may be synonymous, when no modification in the corresponding amino acid occurs, or non-synonymous, when the corresponding amino acid is altered. Synonymous mutations do not have any impact on the function of translated proteins, but may have an impact on the regulation of the corresponding genes or even of other genes, if the mutated sequence is located in a binding site for a regulator factor. Non-synonymous mutations may have an impact on the function or activity of the translated protein as well as on regulation depending on the nature of the mutated sequence.

In particular, mutations in non-coding sequences may be located upstream of the coding sequence (i.e. in the promoter region, in an enhancer, silencer, or insulator region, in a specific transcription factor binding site) or downstream of the coding sequence. Mutations introduced in the promoter region may be in the core promoter, proximal promoter or distal promoter. Mutations may be introduced by site-directed mutagenesis using, e.g., Polymerase Chain Reaction (PCR), by random mutagenesis techniques e.g. via mutagenic agents (Ultra-Violet rays or chemical agents like nitrosoguanidine (NTG) or ethylmethanesulfonate (EMS)), DNA shuffling, error-prone PCR, or using culture conditions that apply a specific stress on the microorganism and induce mutagenesis. The insertion of one or more supplementary nucleotide(s) in the region located upstream of a gene can notably modulate gene expression.

A particular way of modulating endogenous gene expression is to exchange the endogenous promoter of a gene (e.g., wild-type promoter) with a stronger or weaker promoter to upregulate or downregulate expression of the endogenous gene. The promoter may be endogenous (i.e. originating from the same species) or exogenous (i.e. originating from a different species). It is well within the ability of the person skilled in the art to select an appropriate promoter for modulating the expression of an endogenous gene. Such a promoter be, for example, a Ptrc, Ptac, or Plac promoter, or the PR or PL lambda promoters. The promoters may be "inducible" by a particular compound or by specific external conditions, such as temperature or light.

A particular way of modulating endogenous protein activity is to introduce nonsynonymous mutations in the coding sequence of the corresponding gene, e.g. according to any of the methods described above. A non-synonymous amino acid mutation that is present in a transcription factor may notably alter binding affinity of the transcription factor toward a cis-element, alter ligand binding to the transcription factor, etc.

A microorganism may also be genetically modified to express one or more exogenous (i.e. heterologous) genes so as to express or overexpress the corresponding gene product (e.g. an enzyme). An "exogenous" or "heterologous" gene as used herein refers to a gene encoding a protein or polypeptide that is introduced into a microorganism in which said gene does not naturally occur. A "heterologous gene" as used herein also refers to a gene that was endogenous to a microorganism (i.e. present in the microorganism prior to any genetic modification) but that, when introduced into the microorganism, is not introduced at the location where the endogenous gene is/was located. More particularly, the heterologous gene may be an endogenous gene in cases where expression of endogenous gene itself in the microorganism is reduced as compared to the microorganism in which the gene naturally occurs (e.g. due to a mutation, a complete or partial deletion of the gene, a modification in the transcriptional regulation of the gene, etc.). In particular, the endogenous gene may no longer be expressed or may be expressed at very low levels. The exogenous gene may be directly integrated into the chromosome of the microorganism, or be expressed extra-chromosomally within the microorganism by plasmids or vectors. For successful expression, exogenous gene(s) must be introduced into the microorganism with all of the regulatory elements necessary for their expression or be introduced into a microorganism that already comprises all of the regulatory elements necessary for their expression. The genetic modification or transformation of microorganisms with one or more exogenous genes is a routine task for those skilled in the art.

One or more copies of a given exogenous gene can be introduced on a chromosome by methods well-known in the art, such as by genetic recombination. When a gene is expressed extra-chromosomally, it can be carried by a plasmid or a vector. Different types of plasmid are notably available, which may differ in respect to their origin of replication and/or their copy number in the cell. For example, a microorganism transformed by a plasmid can contain 1 to 5 copies of the plasmid, about 20 copies, or even up to 500 copies, depending on the nature of the selected plasmid. A variety of plasmids having different origins of replication and/or copy numbers are well-known in the art and can be easily selected by the skilled person for such purposes, including, e.g., pTrc, pACYC184, pBR322, pUC18, pUC19, pKC30, pRep4, pHS1, pHS2, or pPLc236.

It should be understood that, in the context of the present invention, when an exogenous gene encoding a protein of interest is expressed in a microorganism, a synthetic version of this gene may preferably be constructed by replacing non-preferred codons or less preferred codons with preferred codons of said microorganism which encode the same amino acid. Indeed, it is well-known in the art that codon usage varies between microorganism species, and that this may impact the recombinant expression level of the protein of interest. To overcome this issue, codon optimization methods have been developed, and are extensively described by Graf *et al.* (2000), Deml *et al.* (2001) and Davis & Olsen (2011). Several software programs have notably been developed for codon optimization determination such as the GeneOptimizer® software (Lifetechnologies) or the OptimumGene™ software of (GenScript). In other words, the exogenous gene encoding a protein of interest is preferably codon-optimized for expression in the microorganism. As a particular example, the heterologous *alaD* gene may be codon optimized for expression in a microorganism such as *E. coli.*

The terms "expressing," "overexpressing," or "overexpression" of a protein of interest, such as an enzyme, refer herein to an increase in the expression level and/or activity of said protein in a microorganism, as compared to the corresponding parent microorganism that does not comprise the modification(s) present in the genetically modified microorganism. In some cases, the level of expression may be similar to that of the parent microorganism. In other cases, the level of expression may be superior to that of the parent microorganism. In cases where a parent microorganism does not comprise the protein of interest, the term "expression" or "overexpression" refers to the presence of the protein of interest, as compared to its absence in the parent microorganism.

In contrast, the terms "attenuating" or "attenuation" of a protein of interest refer to a decrease in the expression level and/or activity of said protein in a microorganism, as compared to the parent microorganism. The attenuation of expression can notably be due to either the exchange of the wild-type promoter for a weaker natural or synthetic promoter or the use of an agent reducing gene expression, such as antisense RNA or interfering RNA (RNAi), and more particularly small interfering RNAs (siRNAs) or short hairpin RNAs (shRNAs). Promoter exchange may notably be achieved by the technique of homologous recombination (Datsenko & Wanner, 2000). The complete attenuation of the expression level and/or activity of a protein of interest means that expression and/or activity is abolished; thus, the expression level of said protein is null. The complete attenuation of the expression level and/or activity of a protein of interest may be due to the complete suppression of the expression of a gene. This suppression can be either an inhibition of the expression of the gene, a deletion of all or part of the promoter region necessary for expression of the gene, or a deletion of all or part of the coding region of the gene. A deleted gene can notably be replaced by a selection marker gene that facilitates the identification, isolation and purification of the modified microorganism. As a non-limiting example, suppression of gene expression may be achieved by the technique of homologous recombination, which is well-known to the person skilled in the art (Datsenko & Wanner, 2000).

Modulating the expression level of one or more proteins may thus occur by altering the expression of one or more endogenous genes that encode said protein within the microorganism as described above or by introducing one or more heterologous genes that encode said protein into the microorganism.

The term "expression level" as used herein, refers to the amount (e.g. relative amount, concentration) of a protein of interest (or of the gene encoding said protein) expressed in a microorganism, which is measurable by methods well-known in the art. The level of gene expression can be measured by various known methods including Northern blotting, quantitative RT-PCR, and the like. Alternatively, the level of expression of the protein coded by said gene may be measured, for example by SDS-PAGE, HPLC, LC/MS and other quantitative proteomic techniques (Bantscheff et al., 2007), or, when antibodies against said protein are available, by Western Blot-Immunoblot (Burnette, 1981), Enzyme-linked immunosorbent assay (e.g. ELISA) (Engvall and Perlman, 1971), protein immunoprecipitation, immunoelectrophoresis, and the like. The copy number of an expressed gene can be quantified, for example, by restricting chromosomal DNA followed by Southern blotting using a probe based on the gene sequence, fluorescence in situ hybridization (FISH), RT-qPCR, and the like.

Overexpression of a given gene or the corresponding protein may be verified by comparing the expression level of said gene or protein in the genetically modified organism to the expression level of the same gene or protein in a control microorganism that does not have the genetic modification (i.e. the parental microorganism).

The terms "activity" or "function" as used herein in the context of an enzyme designate the reaction that is catalyzed by said enzyme for converting its corresponding substrate(s) into another molecule(s) (i.e. product(s)). As is well-known in the art, the activity of an enzyme may be assessed by measuring its catalytic efficiency and/or Michaelis constant. Such an assessment is described for example in Segel, 1993, in particular on pages 44-54 and 100-112, incorporated herein by reference.

The term "transcription factor" as used herein refers to a protein, more specifically the "leucine regulatory protein" or "Lrp," that possesses a biological function including regulation of transcription of genes. The Lrp transcription factor possesses a DNA-binding domain that allows it to bind a specific sequence of DNA such as an enhancer element or promoter sequence. Binding may in some cases be dependent on the presence or absence of an allosteric binding protein, such as leucine. Upon binding the enhancer or promoter element, the transcription factor may aide in initiation of transcription, for example, by stabilizing transcription initiation complex formation and/or activity. Transcription factors may also bind to regulatory DNA sequences, such as enhancer sequences, that may be many hundreds of base pairs downstream or upstream from the transcribed gene. Transcription factors may modulate transcription either alone or in combination with other proteins, i.e. by forming an activation complex that may aide in recruiting RNA polymerase and related proteins to the transcription initiation start site.

"Transcription factor activity" as used herein refers to the capacity of a transcription factor to modulate expression of one or more genes by increasing or decreasing the rate of their transcription. The transcription factor may act directly on the gene, e.g. by binding a cis element present in the promoter of the gene or indirectly, e.g. by modulating expression of another element or transcription factor which in turn regulates the transcription of the gene. Transcription factor activity may be evaluated, for example, by electrophoretic gel shift assay or DNA footprinting in cases where the transcription factor directly binds to a cis-element. Reporter assays using e.g. β-galactosidase, luciferase, or GFP (Green Fluorescent Protein) as a reporter gene may also be used to determine transcription factor activity. More particularly, in the context of the present invention, Lrp transcription factor activity and/or expression is reduced as compared to that of a parent microorganism. In turn, the inventors have found that this surprisingly reduces *alaE* gene expression (data not shown). In other words, the microorganism provided herein notably has reduced *alaE* gene expression as compared to that of the parent microorganism.

The microorganism genetically modified for the production of alanine provided herein expresses a heterologous gene coding an enzyme having alanine dehydrogenase activity and has reduced Lrp transcription factor activity and/or expression. Indeed, the inventors have surprisingly shown that the above genetic modifications improve alanine titer, production, and yield, as compared to a parent microorganism that does not comprise these modifications. Improved alanine production in this microorganism is particularly surprising as reduced Lrp transcription factor activity and/or expression reduces the expression level of the *alaE* gene coding the L-alanine exporter. Indeed, *alaE* is generally overexpressed in microorganisms modified for the production of L-alanine.

Preferably, the microorganism genetically modified for the production of alanine provided herein expresses a heterologous *alaD* gene coding an alanine dehydrogenase and has reduced Lrp transcription factor activity and/or expression. More specifically, the microorganism genetically modified for the production of alanine provided herein expresses a heterologous *alaD* gene coding an alanine dehydrogenase and has reduced Lrp transcription factor activity and/or expression as compared to a parent microorganism.

Preferably, the Lrp protein itself is attenuated. Preferably, the microorganism of the invention comprises an *lrp* gene coding for a mutated Lrp protein (otherwise referred to herein as an "Lrp* mutant" protein) having reduced transcription factor activity. The skilled person may readily determine if a given Lrp* protein has reduced transcription factor activity.

As a non-limiting example, the Lrp* mutant may comprise one, two, three or more amino acid substitutions and one, two or more amino acid insertions. When an amino acid in a protein is replaced by another amino acid, the total number of amino acids in the protein does not change. In contrast, when one or more amino acids are inserted, the number of amino acids in the protein increases accordingly. The position of an insertion is the position at which the amino acid(s) are inserted with respect to the unmodified sequence (i.e. the corresponding position).

Corresponding positions can notably be determined by those skilled in the art using manual alignment or by using an alignment program (e.g., BLASTP). Corresponding positions can also be based on structural alignments, for example by using computer-simulated alignments of protein structures. The fact that an amino acid of a polypeptide corresponds to an amino acid in the disclosed sequence means that when the polypeptide and the disclosed sequence are aligned, a standard alignment calculation method such as a GAP calculation method is used. A corresponding amino acid may notably be identified when conserved amino acids are aligned such that the sequences have maximized identity or homology. As used herein, "in a corresponding position" refers to a position of interest in a nucleic acid molecule or protein (i.e. nucleotide base or amino acid residue number) relative to a position in a reference nucleic acid molecule or protein. Positions of interest relative to positions in reference proteins can be, for example, allelic variants, heterologous proteins, amino acid sequences of the same protein in other species, etc. Corresponding positions can be determined by comparing and aligning sequences such that the number of paired nucleotides or amino acid residues is maximized. For example, identity between sequences may be greater than 95%, 96%, 97%, 98%, or more particularly greater than 99%. The position of interest is then given the number assigned in the sequence of the reference nucleic acid molecule or polypeptide. The skilled person will recognize that, in an Lrp* mutant polypeptide, amino acid residue 1 of the modified polypeptide corresponds to amino acid residue 1 of the unmodified Lrp polypeptide (as provided in SEQ ID NO: 1). Indeed, SEQ ID NO: 1 as provided herein is the reference sequence for the Lrp protein. Similarly, SEQ ID NO: 2 as provided herein is the reference sequence for the *lrp* gene.

Preferably, said amino acid mutations are located in the protein C-terminal domain corresponding to the RAM (regulation of amino-acid metabolism) domain. Said substitutions may notably be selected from among L108F, L74F, and F113C. The positions of the amino acid residues indicated correspond to those provided in SEQ ID NO: 1. In the case of L108F and L74F, the substituting amino acid is not limited to phenylalanine but may be any amino acid with an uncharged residue. As a non-limiting example, instead of phenylalanine, L108 and/or L74 may be replaced with tyrosine, tryptophan, alanine, isoleucine, or valine. Said insertion may correspond to 123PD. In the context of the present invention, the insertion "123PD" corresponds to an insertion of two amino acids after position 123. In other words, the amino acids at positions 124 and 125 of the corresponding Lrp* are 124P and 125D, the amino acids at or after position 124 of SEQ ID NO: 1 have been displaced by two amino acid residues.

According to a preferred embodiment, the microorganism comprises an Lrp* mutant wherein the Lrp* mutant comprises at least one mutation selected from the group consisting of L108F, L74F, F113C, and 123PD, wherein the positions of the amino acid residues correspond to those provided in SEQ ID NO: 1. Thus, according to a preferred embodiment, the microorganism comprises an Lrp* mutant having the sequence of SEQ ID NO: 3, 5, 7, or 9. According to a preferred embodiment, the microorganism comprises a gene coding for an Lrp* mutant having the sequence of SEQ ID NO: 4, 6, 8, or 10.

Alternatively, the microorganism comprises an attenuation in the expression of the *lrp* gene. More particularly, said attenuation results from at least a partial deletion of the *lrp* gene or a complete deletion. The term "partial deletion" as used herein refers to the loss of at least 0.5%, 1%, 5%, 10%, 20%, 40%, 50%, 60%, 70%, 80%, 90% or 99% of the nucleotides forming the nucleotide sequence of a gene. The term "complete deletion" as used herein refers to the loss of 100% of the nucleotides forming the nucleotide sequence of a gene.

According to a preferred embodiment, the microorganism comprises at least a partial deletion of the *lrp* gene, preferably a complete deletion of the *lrp* gene.

As mentioned above, the microorganism comprises a heterologous enzyme having alanine dehydrogenase activity. Preferably, said enzyme having alanine dehydrogenase activity is encoded by a heterologous *alaD* gene. Said heterologous enzyme having alanine dehydrogenase activity or said heterologous *alaD* gene coding an alanine dehydrogenase may be derived from a microorganism of one of the following genera: *Bacillus, Geobacillus, Klebsiella, Archaeoglobus, Lysinibacillus, Thermus, Mycobacterium,* or *Phormidium.* More particularly, said *Bacillus* may be *Bacillus subtilis,* said *Geobacillus* may be *Geobacillus stearothermophilus,* said *Klebsiella* may be *Klebsiella aerogenes,* said *Archaeoglobus* may be *Archaeoglobus fulgidus,* said *Lysinibacillus* may be *Lysinibacillus sphaericus,* said *Thermus* may be *Thermus thermophilus,* said *Mycobacterium* may be *Mycobacterium tuberculosis,* or said *Phormidium* may be *Phormidium lapideum.* According to a preferred embodiment, the microorganism expresses an *alaD* gene of *Geobacillus stearothermophilus, Klebsiella aerogenes* or *Archaeoglobus fulgidus.* According to a preferred embodiment, the microorganism expresses an *alaD* coding for an alanine dehydrogenase of SEQ ID NO: 11, 13, 15, 17, 19, 21, 23, 25, 27 or 29, or a functional fragment or functional variant thereof. More preferably, the *alaD* gene codes an alanine dehydrogenase of SEQ ID NO:11, 13, 15, 17, 19, 21, 23, 25, 27 or 29, even more preferably, the *alaD* gene codes the alanine dehydrogenase of SEQ ID NO:15, 17, 19, 23, or 27. Preferably, said heterologous *alaD* gene coding an alanine dehydrogenase is selected from among the genes having the sequence of SEQ ID NO: 12, 14, 16, 18, 20, 22, 24, 26, 28 and 30. More preferably, said said heterologous *alaD* gene coding an alanine dehydrogenase is selected from among the genes having the sequence of SEQ ID NO: 16, 18, 20, 24, and 28.

The term "functional fragment" of a protein of reference having a biological activity of interest (e.g. of an enzyme having alanine dehydrogenase activity), as used herein refers to parts of the amino acid sequence of an enzyme, said parts comprising at least all the regions essential for exhibiting the biological activity of said protein. These parts of sequences can be of various lengths, provided that the biological activity of the amino acid sequence of reference is retained by said parts. In other words, the functional fragments of the enzymes provided herein are enzymatically active.

"Functional variants" of an enzyme described herein (e.g. of an enzyme having alanine dehydrogenase activity) include, but are not limited to, enzymes having amino acid sequences which are at least 60% identical after alignment to the amino acid sequence encoding the corresponding reference enzyme. According to the present invention, the variant preferably has at least 70% 80%, 85%, 90%, 95%, 96%, 97%, 98%, 99%, or 100% amino acid sequence identity to the protein described herein (e.g. an AlaD protein). Thus, the enzyme having alanine dehydrogenase activity preferably has at least 70% 80%, 85%, 90%, 95%, 96%, 97%, 98%, 99%, or 100% sequence identity to the amino acid sequence of SEQ ID NO:11, 13, 15, 17, 19, 21, 23, 25, 27 or 29. More preferably, the gene encoding the enzyme having alanine dehydrogenase activity has at least 70%, 80%, 85%, 90%, 95%, 96%, 97%, 98%, 99%, or 100% sequence identity to the nucleotide sequence of SEQ ID NO:12, 14, 16, 18, 20, 22, 24, 26, 28 or 30. As a non-limiting example, means of determining sequence identity are further provided below.

In addition to the modifications described above, the genetically modified microorganism may comprise one or more additional modifications among those described below. Said modifications are advantageous as they may notably further improve alanine production, titer, and/or yield. One or more of said modifications may notably promote alanine synthesis, inhibit the use of alanine as a substrate in downstream metabolic pathways, promote stable accumulation of alanine or inhibit toxic accumulation of alanine in the microorganism.

In particular, said microorganism may further comprise the overexpression of the aromatic amino acid exporter YddG. Preferably, the amino acid sequence YddG has at least 80% identity, more preferably at least 90% identity, even more preferably at least 95%, 96%, 97%, 98%, or 99% identity, most preferably 100% identity, with the sequence of SEQ ID NO: 31. YddG may notably be overexpressed via the introduction of an exogenous *yddG* gene into the microorganism (e.g. on a plasmid). Alternatively or in addition, the endogenous *yddG* gene may be overexpressed by one or more modifications to said gene or corresponding promoter region, preferably as described herein. Preferably, the *yddG* gene coding for said exporter is overexpressed. Preferably, said *yddG* gene encodes the YddG protein having the sequence of SEQ ID NO: 31. More preferably, said *yddG* gene has a sequence having at least 80% sequence identity, more preferably at least 90% identity, even more preferably at least 95%, 96%, 97%, 98%, or 99% identity, most preferably 100% identity with the sequence of SEQ ID NO: 32.

According to a preferred embodiment, the microorganism further comprises an overexpression of the *yddG* gene, more preferably wherein a plasmid comprising a *yddG* gene is introduced into said microorganism.

The microorganism may further comprise expression of the L-alanine exporter AlaE. Indeed, in the microorganism of the invention, AlaE expression is significantly inhibited due to reduced Lrp transcription factor activity and/or expression. AlaE expression is preferably inferior or equal to that present in the corresponding parent microorganism (i.e. which does not comprise the genetic modifications described herein, in particular which does not comprise reduced Lrp transcription factor activity and/or expression and which does not comprise a heterologous *alaD* gene coding an alanine dehydrogenase according to any of the embodiments provided herein). In other words, AlaE expression is at least partially, preferably completely, restored to levels observed in the corresponding parent microorganism. Preferably the level of AlaE expression is similar to that of the corresponding parent microorganism. Preferably the level of AlaE expression is not superior to that of the corresponding parent microorganism. Preferably, the amino acid sequence of AlaE has at least 80% identity, more preferably at least 90% identity, even more preferably at least 95% identity, most preferably 100% identity, with the sequence of SEQ ID NO: 33.

AlaE expression may notably be restored by modifying the *alaE* promoter or by increasing the number of copies of the *alaE* gene present in the microorganism, in particular according to one of the methods described herein.

Thus, according to a preferred embodiment, the microorganism further comprises expression of an *alaE* gene coding an L-alanine exporter at a level similar to that of the corresponding parent microorganism, preferably by modifying the *alaE* promoter or by increasing the number of copies of the *alaE* gene present in the microorganism. Preferably, *alaE* has at least 80% identity, more preferably at least 90% identity, even more preferably at least 95% identity, most preferably 100% identity, with the sequence of SEQ ID NO: 34.

According to a particularly preferred embodiment, the microorganism provided herein further comprises both an overexpression of the *yddG* gene and expression of an *alaE* gene coding an L-alanine exporter at a level similar to that of the corresponding parent microorganism, preferably by modifying the *alaE* promoter or by increasing the number of copies of the *alaE* gene present in the microorganism.

The microorganism may further comprises the attenuation of at least one enzyme selected from among the acetate kinase AckA, the aldehyde-alcohol dehydrogenase AdhE, the fumarate reductase FrdABCD, comprising a flavoprotein subunit FrdA, a fumarate reductase iron-sulfur protein FrdB, and the fumarate reductase membrane proteins FrdC and FrdD, the D-lactate dehydrogenase LdhA, the methylglyoxal reductase MgsA, the pyruvate formate-lyase activating enzyme PflA, the inactive pyruvate formate-lyase PflB, and the phosphate acetyltransferase Pta. Preferably, when the activity of at least one of the above enzymes is attenuated, said activity is completely attenuated. Said complete attenuation is preferably due to a partial or complete deletion of the gene coding for said enzyme, even more preferably a complete deletion of the gene coding for said enzyme.

Thus, according to a preferred embodiment of the invention, the microorganism further comprises the deletion of at least one of the following genes: *ackA, adhE, frdABCD, ldhA, mgsA, pflAB* and *pta,* or any combination thereof. More preferably, the microorganism further comprises the deletion of the genes: *ackA, adhE, frdABCD, ldhA, mgsA, pflAB* and *pta.* Said genes are notably endogenous in *E. coli.* Preferably, said *ackA-pta* genes have the sequence having at least 80% sequence identity, more preferably at least 90% identity, even more preferably at least 95% identity, most preferably 100% identity, with the sequence of SEQ ID NO: 35 and the sequence of SEQ ID NO: 36, respectively. Preferably, said *adhE* gene has a sequence having at least 80% sequence identity, more preferably at least 90% identity, even more preferably at least 95% identity, most preferably 100% identity, with the sequence of SEQ ID NO: 37. Preferably, said *frdABCD* genes have the sequences having at least 80% sequence identity, more preferably at least 90% identity, even more preferably at least 95% identity, most preferably 100% identity, with the sequences of SEQ ID NOs: 38, 39, 40, and 41, respectively. Preferably, said *ldhA* gene has a sequence having at least 80% sequence identity, more preferably at least 90% identity, even more preferably at least 95% identity, most preferably 100% identity, with the sequence of SEQ ID NO: 42. Preferably, said *mgsA* gene has a sequence having at least 80% sequence identity, more preferably at least 90% identity, even more preferably at least 95% identity, most preferably 100% identity, with the sequence of SEQ ID NO: 43. Preferably, said *pflAB* genes have the sequences having at least 80% sequence identity, more preferably at least 90% identity, even more preferably at least 95% identity, most preferably 100% identity, with the sequences of SEQ ID NOs: 44 and 45. Preferably, said deletion is a complete deletion of the coding region of each of said genes.

Preferably, the microorganism comprises an attenuation of the inner membrane protein CycA which mediates the uptake of D-serine, D-alanine, and glycine and/or the DadX alanine racemase. Said genes are notably endogenous in *E. coli.* Preferably, expression of CycA and/or DadX is attenuated, more preferably completely attenuated. Preferably, the *cycA* and/or *dadX* gene is attenuated, preferably due to a partial or complete deletion of the gene(s) coding for said protein(s), more preferably a complete deletion of the gene(s). Preferably, the *cycA* gene has a sequence having at least 80% sequence identity, more preferably at least 90% identity, even more preferably at least 95% identity, most preferably 100% identity, with the sequence of SEQ ID NO: 46. Preferably, the *dadX* gene has a sequence having at least 80% sequence identity, more preferably at least 90% identity, even more preferably at least 95% identity, most preferably 100% identity, with the sequence of SEQ ID NO: 47.

In a further aspect, when the microorganism as described herein is unable to use sucrose as a carbon source, said microorganism is modified to be able to use sucrose as a carbon source. Preferably, proteins involved in the import and metabolism of sucrose are overexpressed. Preferably, the following proteins are overexpressed:

- CscB sucrose permease, CscA sucrose hydrolase, CscK fructokinase, and CscR *csc*-specific repressor, or
- *ScrA* Enzyme II of the phosphoenolpyruvate-dependent phosphotransferase system and, said *ScrK* gene encodes ATP-dependent fructokinase, said *ScrB* sucrose 6-phosphate hydrolase (invertase), said *ScrY* sucrose porine, ScrR sucrose operon repressor.

Preferably, genes coding for said proteins are overexpressed according to one of the methods provided herein. Preferably, the *E. coli* microorganism overexpresses:

- the heterologous *cscBKAR* genes of *E. coli* EC3132, or
- the heterologous *scrKYABR* genes of *Salmonella sp.*

Genes and proteins are identified herein using the denominations of the corresponding genes in *E. coli* (e.g. *E. coli* K12 MG1655 having the Genbank accession number U00096.3) unless otherwise specified. However, in some cases use of these denominations has a more general meaning according to the invention and covers all of the corresponding genes and proteins in microorganisms. This is notably the case for the genes and proteins described herein that are not endogenous to the microorganism of the invention (i.e. that are heterologous), such as AlaD. As a particular example, and as indicated above, functional variants of AlaD, are comprised herein, as are mutants and functional fragments thereof. Particular aspects are further detailed below.

PFAM (protein family database of alignments and hidden Markov models; http://www.sanger.ac.uk/Software/Pfam/) represents a large collection of protein sequence alignments. Each PFAM makes it possible to visualize multiple alignments, see protein domains, evaluate distribution among organisms, gain access to other databases, and visualize known protein structures.

COGs (clusters of orthologous groups of proteins; http://www.ncbi.nlm.nih.gov/COG/) are obtained by comparing protein sequences from 43 fully sequenced genomes representing 30 major phylogenic lines. Each COG is defined from at least three lines, which permits the identification of former conserved domains.

The means of identifying similar sequences and their percent identities are well-known to those skilled in the art, and include in particular the BLAST programs, which can be used from the website http://www.ncbi.nlm.nih.gov/BLAST/ with the default parameters indicated on that website. The sequences obtained can then be exploited (e.g., aligned) using, for example, the programs CLUSTALW (http://www.ebi.ac.uk/clustalw/) or MULTALIN (http://prodes.toulouse.inra.fr/multalin/cgi-bin/multalin.pl), with the default parameters indicated on those websites.

Using the references given on GenBank for known genes, the person skilled in the art is able to determine the equivalent genes in other organisms, bacterial strains, yeasts, fungi, mammals, plants, etc. This routine work is advantageously done using consensus sequences that can be determined by carrying out sequence alignments with genes derived from other microorganisms, and designing degenerate probes to clone the corresponding gene in another organism. These routine methods of molecular biology are well known to those skilled in the art, and are described, e.g., in Sambrook and Russell, 2001.

Sequence identity between amino acid sequences can be determined by comparing a position in each of the sequences which may be aligned for the purposes of comparison. When a position in the compared sequences is occupied by the same amino acid, then the sequences are identical at that position. A degree of sequence identity between proteins is a function of the number of identical amino acid residues at positions shared by the sequences of said proteins.

As a non-limiting example, to determine the percentage of identity between two amino acid sequences, the sequences are aligned for optimal comparison. For example, gaps can be introduced in the sequence of a first amino acid sequence for optimal alignment with the second amino acid sequence. The amino acid residues at corresponding amino acid positions are then compared. When a position in the first sequence is occupied by the same amino acid residue as the corresponding position in the second sequence, the molecules are identical at that position.

The percentage of identity between the two sequences is a function of the number of identical positions shared by the sequences. Hence % identity = number of identical positions / total number of overlapping positions $\chi$ 100.

Optimal alignment of sequences may be conducted by the global alignment algorithm of Needleman and Wunsch (1972), by computerized implementations of this algorithm (such as CLUSTAL W) or by visual inspection. The best alignment (i.e., resulting in the highest percentage of identity between the compared sequences) generated by the various methods is selected.

In other words, the percentage of sequence identity is calculated by comparing two optimally aligned sequences, determining the number of positions at which the identical amino acid occurs in both sequences to yield the number of matched positions, dividing the number of matched positions by the total number of positions and multiplying the result by 100 to yield the percentage of sequence identity.

The above definitions and preferred embodiments related to the functional fragments and functional variants of proteins apply *mutatis mutandis* to nucleotide sequences, such as genes, encoding a protein of interest (i.e. an enzyme having alanine dehydrogenase activity).

A second object of the invention relates to a method for the production of alanine using the microorganism described herein. Said method comprises the steps of:

a) culturing a microorganism genetically modified for the production of alanine as described herein in an appropriate culture medium comprising a source of carbon, and

b) recovering alanine from the culture medium.

More specifically, the invention relates to a method for the improved fermentative production of alanine using the microorganism described herein. According to the invention, the terms "fermentative process," "fermentative production," "fermentation," or "culture" are used interchangeably to denote the growth of microorganism. This growth is generally conducted in fermenters with an appropriate growth medium adapted to the microorganism being used.

An "appropriate culture medium" designates a medium (e.g., a sterile, liquid media) comprising nutrients essential or beneficial to the maintenance and/or growth of the cell such as carbon sources or carbon substrates, nitrogen sources, for example, peptone, yeast extracts, meat extracts, malt extracts, urea, ammonium sulfate, ammonium chloride, ammonium nitrate and ammonium phosphate; phosphorus sources, for example, monopotassium phosphate or dipotassium phosphate; trace elements (e.g., metal salts), for example magnesium salts, cobalt salts and/or manganese salts; as well as growth factors such as amino acids and vitamins. In particular, the inorganic culture medium for *E. coli* can be of identical or similar composition to an M9 medium (Anderson, 1946), an M63 medium (Miller, 1992) or a medium such as defined by Schaefer et al. (1999).

The term "source of carbon," "carbon source," or "carbon substrate" according to the present invention refers to any carbon source capable of being metabolized by a microorganism wherein the substrate contains at least one carbon atom. According to the present invention, said source of carbon is preferably at least one carbohydrate, and in some cases a mixture of at least two carbohydrates. $CO_2$ is not a carbohydrate because it does not contain hydrogen.

The term "carbohydrate" refers to any carbon source capable of being metabolized by a microorganism and containing at least one carbon atom, two atoms of hydrogen and one atom of oxygen. The one or more carbohydrates may be selected from among the group consisting of: monosaccharides such as glucose, fructose, mannose, xylose, arabinose, galactose and the like, disaccharides such as sucrose, cellobiose, maltose, lactose and the like, oligosaccharides such as raffinose, stacchyose, maltodextrins and the like, polysaccharides such as cellulose, hemicellulose, starch and the like, methanol, formaldehyde and glycerol. Preferred carbon sources are arabinose, fructose, galactose, glucose, lactose, maltose, sucrose, xylose or any combination thereof, more preferably glucose.

The term "recovering" as used herein designates the process of separating or isolating the produced alanine by using conventional laboratory techniques known to the person skilled in the art. Recovering alanine according to step b) of the method described herein may comprise a step of filtration, desalination, cation exchange, liquid extraction, crystallization, or distillation, or combinations thereof. Alanine may be recovered from both culture medium and microorganisms, or from only one or the other. Preferably, alanine is recovered from at least the culture medium. The volume of culture medium may be reduced for example via ceramic membrane filtration. Alanine may furthermore be recovered either during culturing of the microorganism by *in situ* product recovery including extractive fermentation, or after fermentation is finished. Microorganisms may notably be removed by passing through a device, preferably through a filter with a cut-off in the range from 5 to 200 kDa, where solid/liquid separation takes place. It is also feasible to employ a centrifuge, a suitable sedimentation device or a combination of these devices, it being especially preferred to first separate at least part of the microorganisms by sedimentation and subsequently to feed the fermentation broth, from which the microorganisms have been at least partially removed, to ultrafiltration or to a centrifugation device. After the microorganisms have been removed, alanine present in the remaining culture medium may be recovered. Alanine may be recovered from microorganisms separately. Recovery of alanine from microorganism may notably involve lysis or disruption by heating to induce alanine release from microorganisms.

Those skilled in the art are able to define the culture conditions for the microorganisms according to the invention. In particular the bacteria are fermented at a temperature between 20°C and 55°C, preferably between 25°C and 40°C, more preferably between about 30°C to 37°C, even more preferably about 37°C.

This process can be carried out either in a batch process, in a fed-batch process or in a continuous process. It can be carried out under aerobic, micro-aerobic or anaerobic conditions, or a combination thereof (for example, aerobic conditions followed by anaerobic conditions).

"Under aerobic conditions" means that oxygen is provided to the culture by dissolving the gas into the liquid phase. This could be obtained by (1) sparging oxygen containing gas (e.g. air) into the liquid phase or (2) shaking the vessel containing the culture medium in order to transfer the oxygen contained in the head space into the liquid phase. The main advantage of the fermentation under aerobic conditions is that the presence of oxygen as an electron acceptor improves the capacity of the strain to produce more energy under the form of ATP for cellular processes. Therefore, the strain has its general metabolism improved.

Micro-aerobic conditions are defined as culture conditions wherein low percentages of oxygen (e.g. using a mixture of gas containing between 0.1 and 10% of oxygen, completed to 100% with nitrogen), is dissolved into the liquid phase.

Anaerobic conditions are defined as culture conditions wherein no oxygen is provided to the culture medium. Strictly anaerobic conditions are obtained by sparging an inert gas like nitrogen into the culture medium to remove traces of other gas. Nitrate can be used as an electron acceptor to improve ATP production by the strain and improve its metabolism.

The production of alanine by the microorganism in the culture broth can be determined unambiguously by standard analytical means known by those skilled in the art. As a non-limiting example, alanine may be quantified using isocratic HPLC (Pleissner et al., 2011) or nuclear magnetic resonance.

In a further aspect, the method described herein further comprises a step c) of purifying alanine. Alanine may be purified by using conventional laboratory techniques known to the skilled person, such as concentration, filtration, ion-exchange, or crystallization methods, or combinations thereof. Alanine may be further purified by using conventional laboratory techniques known to the skilled person, such as filtration and/or crystallization. Methods of recovering and/or purifying alanine are notably described in CN103965064A and CN103965064A. As an example, alanine may be purified after being mixed with an organic solvent.

## Examples

The present invention is further defined in the following examples. It should be understood that these examples, while indicating preferred embodiments of the invention, are given by way of illustration only. From above disclosure and these examples, the person skilled in the art can make various changes of the invention to adapt it to various uses and conditions without modifying the essential means of the invention.

In the examples given below, methods well-known in the art were used to construct *E. coli* strains containing replicating vectors and/or various chromosomal deletions, and substitutions using homologous recombination, as is well-described in Datsenko & Wanner, (2000) for *E. coli*. In the same manner, the use of plasmids or vectors to express or overexpress one or more genes in a recombinant microorganism are well-known by the person skilled in the art. Examples of suitable *E. coli* expression vectors include pTrc, pACYC184, pBR322, pUC18, pUC19, pKC30, pRep4, pHS1, pHS2, pPLc236, etc.

**Chromosomal modifications**. Several protocols have been used in the following examples. Protocol 1 (chromosomal modifications by PCR amplification using oligonucleotides and appropriate genomic DNA as a matrix (that the person skilled in the art will be able to define), homologous recombination and selection of recombinants), protocol 2 (transduction of phage P1) and protocol 3 (antibiotic cassette excision, the resistance genes were removed when necessary) used in this invention have been fully described in patent application EP 2532751 (see in particular example 1 and example 3, points 1.2 and 1.3, incorporated herein by reference). Chromosomal modifications were verified by PCR analysis with appropriate oligonucleotides that the person skilled in the art is able to design.

**Construction of recombinant plasmids.** Recombinant DNA technology is well described in the literature and routinely used by the person skilled in the art. Briefly, DNA fragments were PCR amplified using oligonucleotides and appropriate genomic DNA as a matrix (that the person skilled in the art will be able to define). The DNA fragments and chosen plasmid were digested with compatible restriction enzymes (that the person skilled in the art will be able to define), then ligated and transformed into competent cells. Transformants were analyzed and recombinant plasmids of interest were verified by DNA sequencing. Kanamycin (50 mg/L) and/or chloramphenicol (30 mg/L) was added to the medium when necessary.

**Strain selection based on the expression of genes responsible for antibiotic resistance**. Strains construction required the selection of cells harboring a DNA fragment responsible for a specific antibiotic resistance. To achieve this selection, bacteria were spread on petri dishes containing LB solid medium (10g/L bactopeptone, 5 g/L yeast extract, 5 g/L NaCl and 20 g/L agar). Antibiotics were added when necessary according to the selection marker: chloramphenicol (30 mg/L); kanamycin (50 mg/L); gentamycin (10 mg/L).

**Culture conditions**. Strains that produced substantial amounts of metabolites of interest were subsequently tested under production conditions in 2.5 L fermentors (Pierre Guerin) using a strategy of limiting growth by the feeding rate and glucose concentration in the feeding medium. A 50 mL preculture was grown at 37°C for 16 hours in a mixed medium (10% LB medium with 2.5 $g.L^{-1}$ glucose and 90% B1 minimal medium, Table 1). It was used to inoculate a 1 L culture to an $OD_{600}$ of 1 in PC1 medium. The culture temperature was maintained constant at 37°C and pH was maintained to the working value (6.8) by automatic addition of $NH_4OH$ solution ($NH_4OH$ 28%). The initial agitation rate was set at 150 RPM. After 6 hours of batch phase, the feeding rate (F1 medium) was started at a value of 0.01 $L.h^{-1}$ and kept constant for 30 hours. Then, the feeding rate was gradually increased to a value of 0.05 $L.h^{-1}$. Glucose was limiting for the duration of the culture. Strains were compared after culture for 120 hours.

Table 1: Media composition for the culture of alanine production strains.

| Compound | Concentration (g/L) | |
|---|---|---|
| | **B1** | **F1** |
| Disodium ethylenediaminetetraacetate dihydrate | 0.0088 | 0.0084 |
| Cobalt (II) chloride hexahydrate | 0.0026 | 0.0025 |
| Manganese (II) chloride tetrahydrate | 0.0157 | 0.015 |
| Copper (II) chloride dihydrate | 0.00157 | 0.0015 |
| Boric acid | 0.0031 | 0.003 |
| Sodium molybdate dihydrate | 0.0026 | 0.0025 |
| Zinc acetate dihydrate | 0.0136 | 0.0136 |
| Potassium dihydrogen phosphate | 1.0484 | 1.000 |
| Ammonium sulfate | 0.5242 | 0.5 |
| Magnesium sulfate heptahydrate | 0.5242 | 0.5 |
| Calcium chloride dihydrate | 0.0210 | 0.02 |
| Ferrous sulfate heptahydrate | 0.0105 | 0.01 |
| Thiamine hydrochloride | 0.0121 | 0.0115 |
| Sodium nitrate | 0.6290 | 0.6 |
| Glucose | 50 | 100 |

In these cultures, the alanine yield ($Y_{alanine}$) was expressed as followed:

$$Yalanine\ (\frac{g}{g}) = \frac{alanine\ produced\ (g)}{glucose\ consumed\ (g)} * 100$$

**Amino acid quantification conditions.**

Extracellular amino acids were quantified by HPLC after o-phthalaldehyde/fluorenylmethyl-chloroformate (OPA/FMOC) derivatization and other relevant metabolites were analyzed using HPLC with refractometric detection (organic acids and glucose) and GC-MS after silylation.

**EXAMPLE 1: Strain construction**

To prepare the strain for alanine production, combinations of mutations were introduced into the *E. coli* strain resulting in strain 1: MG1655 *DackA+pta DadhE DldhA DfrdABCD DmgsA DpflAB,* constructed as follows.

To inactivate the *ackA+pta, frdABCD, pflAB* operons and the *adhE, ldhA and mgsA* genes, the homologous recombination strategy was used (according to Protocol 1). The strains retained were designated MG1655 D*ackA+pta*::Gt, MG1655 D*adhE*::Cm, MG1655 D*ldhA*::Km, MG1655 *DfrdABCD*::Gt, MG1655 D*mgsA*::Km and MG1655 *DpflAB*::Cm, where Km, Cm and Gt designate respectively DNA sequences conferring resistance to kanamycin, chloramphenicol and gentamycin. All of these deletions were transferred by P1 phage transduction (according to Protocol 2) into *E. coli* MG1655 and resistance genes were removed according to protocol 3 when necessary, giving rise to strain 1.

To produce alanine, the *alaD* gene from *Geobacillus stearothermophilus* (SEQ ID NO: 20) was cloned under the artificial trc promoter without the operator sequence for IPTG induction, into the pME101VB06 plasmid described in patent application EP 2532751 (see in particular Examples 3-6, incorporated herein by reference). The resulting plasmid, pNX0001, was then transformed into the strain 1 giving rise to strain 2.

To overexpress the alanine exporter, the *alaE* gene from *E. coli* was cloned with its native promoter into the pNX0001 plasmid. The resulting plasmid, pAL0006, was then transformed into strain 2 giving rise to strain 7.

To overexpress the amino acid exporter, the *yddG* gene from *E. coli* was cloned with its native promoter into the pACYC184 plasmid (Chang and Cohen, 1978). The resulting plasmid, pAL0009, was then transformed into strain 2 giving rise to strain 12 and into strain 7 giving rise to strain 17.

The native *lrp* gene was replaced by diverse mutated *lrp* alleles using the homologous recombination strategy (according to Protocol 1) giving rise to strains MG1655 lrp*(L74F)::Cm, MG1655 lrp*(L108F)::Cm, MG1655 lrp*(F113C)::Cm, and MG1655 lrp*(123PD)::Cm (introduction of proline and aspartic acid amino acids after position 123). All of these mutated *lrp* alleles were transferred by P1 phage transduction (according to Protocol 2) into the defined strain and resistance genes were removed according to protocol 3. When necessary, the plasmids were transformed into previous strains giving rise to the new strains described below (Table 2).

Table 2: Strains obtained

| **Lrp* allele** | **into strain 2** | **into strain 7** | **Strains 3 to 6 with pAL0009** | **Strains 8 to 11 with pAL0009** |
|---|---|---|---|---|
| L74F | 3 | 8 | 13 | 18 |
| L108F | 4 | 9 | 14 | 19 |
| F113C | 5 | 10 | 15 | 20 |
| 123PD | 6 | 11 | 16 | 21 |

**EXAMPLE 2: Strains performances with the diverse mutated *lrp* alleles**

Production strains were assessed in culture conditions as previously described.

Table 3: Alanine titer, productivity and yield for the different strains with the mutated *lrp.*

| Strain no. | Titer (g/L) | Prod (g/L/h) | Yield (g/g) |
|---|---|---|---|
| Strain 3 | ++ | +++ | + |
| Strain 4 | ++ | +++ | + |
| Strain 5 | ++ | +++ | + |
| Strain 6 | ++ | +++ | + |

The symbol "+" indicates an increase of a factor up to 2, the symbol "++" an increase by a factor between 2 and 5 and "+++" an increase by a factor greater than 5, as compared to the values of reference strain 2.

As can be seen in table 3, strains 3 to 6 showed an increase in titer, productivity and yield for alanine as compared to strain 2. Performances were increased with the replacement of the native *lrp* by diverse mutated *lrp* alleles. The effect was equivalent with all of the mutated *lrp* alleles tested.

**EXAMPLE 3: Strains performances with overexpression of the amino acid exporter (yddG) in the mutated *Irp* alleles**

Production strains were assessed in culture conditions as previously described.

Table 4: Alanine titer, productivity and yield, for the different strains overexpressing the amino exporter (yddG).

| Strain no. | Titer (g/L) | Prod (g/L/h) | Yield (g/g) |
|---|---|---|---|
| Strain 12 | + | ++ | + |
| Strain 13 | ++ | +++ | + |
| Strain 14 | ++ | +++ | + |
| Strain 15 | ++ | +++ | + |
| Strain 16 | ++ | +++ | + |

The symbol "+" indicates an increase of a factor up to 2, the symbol "++" an increase by a factor between 2 and 5 and "+++" an increase by a factor greater than 5, as compared to the values of reference strain 2.

As can be seen in table 4, overexpression of the amino acid exporter *yddG* in strain 2 (strain 12) improves the performance of the strain. Additionally, with the replacement of the native *Irp* by diverse mutated *Irp* alleles the performance of strains 13 to 16 was further increased. The effect was equivalent with all of the mutated *Irp* alleles tested.

**EXAMPLE 4: Strain performances with overexpression of the alanine exporter (*alaE*) with and without mutated *Irp* alleles**

Production strains were assessed in culture conditions as previously described.

Table 5: Alanine titer, productivity and yield for the different strains overexpressing the alanine exporter (*alaE*).

| Strain no. | Titer (g/L) | Prod (g/L/h) | Yield (g/g) |
|---|---|---|---|
| Strain 7 | + | ++ | + |
| Strain 8 | ++ | +++ | + |
| Strain 9 | ++ | +++ | + |
| Strain 10 | ++ | +++ | + |
| Strain 11 | ++ | +++ | + |

The symbol "+" indicates an increase of a factor up to 2, the symbol "++" an increase by a factor between 2 and 5 and "+++" an increase by a factor greater than 5, as compared to the values of reference strain 2.

As can be seen in table 5, overexpression of the alanine exporter in strain 2 (strain 7) improves the performance of the strain. Additionally, with the replacement of the native *Irp* by diverse mutated *Irp* alleles the performance of strains 8 to 11 was further increased. The effect was equivalent with all of the mutated *Irp* alleles tested.

**EXAMPLE 5: Strain performances with overexpression of the alanine exporter (*alaE*) and the amino acid exporter (*yddG*) with and without the mutated *Irp* alleles**

Production strains were assessed in culture conditions as previously described.

Table 6: Alanine titer, productivity and yield, for the different strains overexpressing the alanine exporter (alaE) and the amino acid exporter (*yddG*).

| Strain no. | Titer (g/L) | Prod (g/L/h) | Yield (g/g) |
|---|---|---|---|
| Strain 17 | ++ | ++ | + |
| Strain 18 | ++ | +++ | + |

(continued)

| Strain no. | Titer (g/L) | Prod (g/L/h) | Yield (g/g) |
|---|---|---|---|
| Strain 19 | ++ | +++ | + |
| Strain 20 | ++ | +++ | + |
| Strain 21 | ++ | +++ | + |

The symbol "+" indicates an increase of a factor up to 2, the symbol "++" an increase by a factor between 2 and 5 and "+++" an increase by a factor greater than 5, as compared to the values of reference strain 2.

As can be seen in table 6, overexpression of the amino acid exporter and the alanine exporter in strain 2 (strain 17) improves the performance of the strain. Additionally, with the replacement of the native *lrp* by diverse mutated *lrp* alleles the performances of strains 18 to 21 were further increased. The effect was equivalent with all of the mutated *lrp* alleles tested.

**References**

- Anderson, (1946), Proc. Natl. Acad. Sci. USA, 32:120-128.
- Bantscheff et al., (2007), Anal Bioanal Chem. 389(4):1017-31.
- Burnette, (1981), Anal Biochem. 112(2):195-203.
- Chang and Cohen, (1978), J Bacteriol. 134(3):1141-56.
- Datsenko KA & Wanner BL, (2000), Proc Natl Acad Sci USA., 97: 6640-6645.
- Davis and Olsen, (2011), Mol. Biol. Evol.; 28(1):211-221.
- Deml, et al., (2001), J. Virol., 75(22): 10991-11001.
- Engvall and Perlman, (1971), Immunochemistry; 8(9):871-4.
- Gmelch, et al., (2019), Sci Rep. 9, 11754.
- Graf et al., (2000), J. Virol.; 74(22): 10/22-10826.
- Ihara et al., (2017), Journal of Bioscience and Bioengineering. 123(4): 444-450
- Leuchtenberger et al., (2005), Appl Microbiol Biotechnol., 69: 1-8.
- Miller, (1992), "A Short Course in Bacterial Genetics: A Laboratory Manual and Handbook for Escherichia coli and Related Bacteria", Cold Spring Harbor Laboratory Press, Cold Spring Harbor, New York.
- Needleman and Wunsch, (1972), J. Mol. Biol., 48(3), 443-453.
- Pleissner et al., (2011), Anal. Chem., 83(1): 175-181.
- Prescott et al., (1999), "Microbiology" 4th Edition, WCB McGraw-Hill.
- Sambrook and Russell, (2001), Molecular Cloning: 3rd edition, Cold Spring Harbor Laboratory Press, NY, Vol 1, 2, 3.
- Sato et al., (1982) Production of L-Alanine from Ammonium Fumarate Using Two Types of Immobilized Microbial Cells. In: Chibata I., Fukui S., Wingard L.B. (eds) Enzyme Engineering. Springer, Boston, MA.
- Schaefer et al., (1999), Anal. Biochem., 270: 88-96.
- Segel (1993), Enzyme kinetics, John Wiley & Sons, pp. 44-54 and 100-112.
- The Expresswire. (2020, June 10). L-Alanine Market Size- Aiming on Current Market Conditions, Competitors, Product Price, Profit, Capacity, Production and Future Forecast to 2026 [Press release]. Retrieved from https://www.theexpresswire.com/pressrelease/2020-L-Alanine-Market-Size-Aiming-on-Current-Market-Conditions-Competitors-Product-Price-Profit-Capacity-Production-and-Future-Forecast-to-2026_11161692
- Wendisch (2014), Curr. Opin. Biotechnol. 30,51-58.
- Zhang et al., (2007), Appl Microbiol Biotechnol. 77(2):355-366.

## Claims

1. Microorganism genetically modified for the production of alanine, wherein the microorganism expresses a heterologous *alaD* gene coding an alanine dehydrogenase and has reduced Lrp transcription factor activity and/or expression.

2. Microorganism of claim 1, wherein the microorganism comprises an *lrp* gene coding for an Lrp* mutant having reduced transcription factor activity.

3. Microorganism of claim 2, wherein the Lrp* mutant comprises at least one mutation selected from the group consisting of L108F, L74F, F113C, and 123PD, wherein the positions of the amino acid residues correspond to those provided in SEQ ID NO: 1.

4. Microorganism of claim 1, comprising at least a partial deletion of the *lrp* gene, preferably a complete deletion of the *lrp* gene.

5. Microorganism of any of claims 1 to 4, wherein the microorganism further comprises an overexpression of the *yddG* gene.

6. Microorganism of any of claims 1 to 5, further comprising expression of an *alaE* gene coding an L-alanine exporter at a level similar to that of the corresponding microorganism which does not comprise the genetic modifications according to any one of claims 1 to 5, preferably by modifying the *alaE* promoter or by increasing the number of copies of the *alaE* gene present in the microorganism.

7. Microorganism of any of claims 1 to 6, wherein the microorganism expresses the *alaD* gene of *Geobacillus stearothermophilus, Klebsiella aerogenes or Archaeoglobus fulgidus.*

8. Microorganism of claim 7, wherein the *alaD* gene codes the alanine dehydrogenase of SEQ ID NO: 15, 17, 19, 23, or 27.

9. Microorganism of any of claims 1 to 8, further comprising a deletion of at least one gene selected from the group consisting of *ackA-pta, ldhA, adhE, frdABCD, mgsA,* and *pflAB.*

10. Microorganism of claim 9, wherein the microorganism comprises the deletion of genes *ackA-pta, ldhA, adhE, frdABCD, mgsA,* and *pflAB.*

11. Microorganism of claim 10 or 11, further comprising a deletion of the *cycA* and/or *dadX* gene(s).

12. Microorganism of any one of claims 1 to 11, wherein said microorganism belongs to the family of bacteria Enterobacteriaceae, Clostridiaceae, Bacillaceae, Streptomycetaceae, or Corynebacteriaceae, or to the family of yeasts Saccharomycetaceae.

13. Microorganism of claim 12, wherein said Enterobacteriaceae bacterium is *Escherichia coli or Klebsiella pneumoniae,* said Clostridiaceae bacterium is *Clostridium acetobutylicum,* said Corynebacteriaceae bacterium is *Corynebacterium glutamicum,* or said Saccharomycetaceae yeast is *Saccharomyces cerevisiae,* more preferably wherein said microorganism is *Escherichia coli.*

14. Method for the production of alanine comprising the steps of:

    a) culturing a microorganism genetically modified for the production of alanine according to any one of claims 1 to 13 in an appropriate culture medium comprising a source of carbon, and
    b) recovering alanine from the culture medium.

15. Method of claim 14, wherein the source of carbon is selected from arabinose, fructose, galactose, glucose, lactose, maltose, sucrose, xylose, and any combination thereof.

Europäisches Patentamt
European Patent Office
Office européen des brevets

# EUROPEAN SEARCH REPORT

Application Number
EP 20 30 5967

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | XUELI ZHANG ET AL: "Production of L-alanine by metabolically engineered Escherichia coli", APPLIED MICROBIOLOGY AND BIOTECHNOLOGY, SPRINGER BERLIN HEIDELBERG, BERLIN/HEIDELBERG , vol. 77, no. 2 1 November 2007 (2007-11-01), pages 355-366, XP002687747, ISSN: 0175-7598, DOI: 10.1007/S00253-007-1170-Y Retrieved from the Internet: URL:http://rd.springer.com/article/10.1007%2Fs00253-007-1170-y | 1,2,5-15 | INV. C12R1/145 C12N9/06 C12P13/06 C12R1/15 C12R1/19 |
| A | * page 358, right-hand column, paragraph 3 - page 365, right-hand column, paragraph 2; figure 1 * ----- | 3,4 | |
| X | WO 2015/028915 A1 (BASF SE [DE]; BASF CHINA CO LTD [CN]) 5 March 2015 (2015-03-05) | 1,2,5,6, 9,14,15 | |
| A | * claims 1-17 * * * ----- | 3,4,7,8, 10-13 | TECHNICAL FIELDS SEARCHED (IPC) C12R C12N C12P |
| A | WO 2016/120326 A1 (UNIV DANMARKS TEKNISKE [DK]) 4 August 2016 (2016-08-04) * page 27, line 6 - page 27, line 26; claims 1-20 * ----- | 1-15 | |

The present search report has been drawn up for all claims

1

| Place of search | Date of completion of the search | Examiner |
|---|---|---|
| Munich | 24 February 2021 | Stoyanov, Borislav |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

**ANNEX TO THE EUROPEAN SEARCH REPORT ON EUROPEAN PATENT APPLICATION NO.** EP 20 30 5967

This annex lists the patent family members relating to the patent documents cited in the above-mentioned European search report. The members are as contained in the European Patent Office EDP file on
The European Patent Office is in no way liable for these particulars which are merely given for the purpose of information.

24-02-2021

| Patent document cited in search report | Publication date | Patent family member(s) | Publication date |
|---|---|---|---|
| WO 2015028915 A1 | 05-03-2015 | CA 2920814 A1 | 05-03-2015 |
| | | CN 105593361 A | 18-05-2016 |
| | | EP 3039121 A1 | 06-07-2016 |
| | | JP 2016529901 A | 29-09-2016 |
| | | KR 20160043973 A | 22-04-2016 |
| | | RU 2016111682 A | 05-10-2017 |
| | | US 2016304917 A1 | 20-10-2016 |
| | | WO 2015028915 A1 | 05-03-2015 |
| WO 2016120326 A1 | 04-08-2016 | BR 112017015874 A2 | 27-03-2018 |
| | | BR 112017015876 A2 | 27-03-2018 |
| | | CA 2975010 A1 | 04-08-2016 |
| | | CA 2975011 A1 | 04-08-2016 |
| | | CN 107406864 A | 28-11-2017 |
| | | CN 107429275 A | 01-12-2017 |
| | | DK 3250700 T3 | 27-07-2020 |
| | | DK 3250701 T3 | 08-02-2021 |
| | | EP 3250700 A1 | 06-12-2017 |
| | | EP 3250701 A1 | 06-12-2017 |
| | | ES 2807448 T3 | 23-02-2021 |
| | | HK 1247247 A1 | 21-09-2018 |
| | | HK 1247248 A1 | 21-09-2018 |
| | | JP 6720194 B2 | 08-07-2020 |
| | | JP 2018503384 A | 08-02-2018 |
| | | JP 2018503385 A | 08-02-2018 |
| | | PL 3250700 T3 | 14-12-2020 |
| | | US 2018010157 A1 | 11-01-2018 |
| | | US 2018016546 A1 | 18-01-2018 |
| | | WO 2016120326 A1 | 04-08-2016 |
| | | WO 2016120345 A1 | 04-08-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 108060114
- WO 2012172822 A
- CN 103965064 A
- EP 2532751 A


### Non-patent literature cited in the description


- **ANDERSON.** *Proc. Natl. Acad. Sci. USA,* 1946, vol. 32, 120-128
- **BANTSCHEFF et al.** *Anal Bioanal Chem.,* 2007, vol. 389 (4), 1017-31
- **BURNETTE.** *Anal Biochem.,* 1981, vol. 112 (2), 195-203
- **CHANG ; COHEN.** *J Bacteriol.,* 1978, vol. 134 (3), 1141-56
- **DATSENKO KA ; WANNER BL.** *Proc Natl Acad Sci USA.,* 2000, vol. 97, 6640-6645
- **DAVIS ; OLSEN.** *Mol. Biol. Evol.,* 2011, vol. 28 (1), 211-221
- **DEML et al.** *J. Virol.,* 2001, vol. 75 (22), 10991-11001
- **ENGVALL ; PERLMAN.** *Immunochemistry,* 1971, vol. 8 (9), 871-4
- **GMELCH et al.** *Sci Rep.,* 2019, vol. 9, 11754
- **GRAF et al.** *J. Virol.,* 2000, vol. 74 (22
- **IHARA et al.** *Journal of Bioscience and Bioengineering,* 2017, vol. 123 (4), 444-450
- **LEUCHTENBERGER et al.** *Appl Microbiol Biotechnol.,* 2005, vol. 69, 1-8
- **MILLER.** A Short Course in Bacterial Genetics: A Laboratory Manual and Handbook for Escherichia coli and Related Bacteria. Cold Spring Harbor Laboratory Press, 1992
- **NEEDLEMAN ; WUNSCH.** *J. Mol. Biol.,* 1972, vol. 48 (3), 443-453
- **PLEISSNER et al.** *Anal. Chem.,* 2011, vol. 83 (1), 175-181
- **PRESCOTT et al.** Microbiology. WCB McGraw-Hill, 1999
- **SAMBROOK ; RUSSELL.** Molecular Cloning. Cold Spring Harbor Laboratory Press, 2001, vol. 1, 2, 3
- Production of L-Alanine from Ammonium Fumarate Using Two Types of Immobilized Microbial Cells. **SATO et al.** Enzyme Engineering. Springer, 1982
- **SCHAEFER et al.** *Anal. Biochem.,* 1999, vol. 270, 88-96
- **SEGEL.** Enzyme kinetics. John Wiley & Sons, 1993, 44-45, 100-112
- L-Alanine Market Size- Aiming on Current Market Conditions, Competitors, Product Price, Profit, Capacity, Production and Future Forecast to 2026 [Press release. *The Expresswire,* 10 June 2020, https://www.theexpresswire.com/pressrelease/2020-L-Alanine-Market-Size-Aiming-on-Current-Market-Conditions-Competitors-Product-Price-Profit-Capacity-Production-and-Future-Forecast-to-2026_11161692
- **WENDISCH.** *Curr. Opin. Biotechnol.,* 2014, vol. 30, 51-58
- **ZHANG et al.** *Appl Microbiol Biotechnol.,* 2007, vol. 77 (2), 355-366